# EUROPEAN PATENT SPECIFICATION

(11) **EP 1 814 606 B1**
(45) Date of publication and mention of the grant of the patent: **22.07.2015**
(21) Application number: 05821230.9
(22) Date of filing: 25.10.2005
(51) Int. Cl.: A61L 24/10, A61L 27/24, A61L 31/04, A61K 6/02

(54) **IMPLANTABLE COLLAGEN COMPOSITIONS**
IMPLANTIERBARE KOLLAGENZUSAMMENSETZUNGEN
COMPOSITIONS DE COLLAGENE IMPLANTABLES

(30) Priority: 10.11.2004 US 985570
(43) Date of publication of application: 08.08.2007
(73) Proprietor: Fibrogen, Inc., San Francisco, CA 94158 (US)
(72) Inventor: OLSEN, David R., Menlo Park, CA 94025 (US); POLAREK, James W., Sausalito, CA 94965 (US); YANG, Chunlin, Bellemead, NJ 08502 (US)
(74) Representative: Goodfellow, Hugh Robin
(86) International application number: PCT/US2005/038521
(87) International publication number: WO 2006/052451

(56) References cited:
- EP-A- 0 713 707
- WO-A-2004/078120
- US-A- 4 582 640
- US-A- 5 116 389
- US-A- 5 428 024
- US-B1- 6 509 031
- ANNAMARI VUORELA ET AL: "Assembly of human prolyl 4-hydroxylase and type III collagen in the yeast Pichia pastoris: formation of a stable enzyme tetramer requires coexpression with collagen and assembly of a stable collagen requires coexpression with prolyl 4-hydroylase" EMBO JOURNAL, OXFORD UNIVERSITY PRESS, SURREY, GB, vol. 16, no. 22, 1997, pages 6702-6712, XP002153732 ISSN: 0261-4189 cited in the application
- S. CLICHE, ET AL.: "Extraction and characterization of collagen with or without telopeptides from chicken skin", POULTRY SCIENCE, vol. 82, 13 October 2002 (2002-10-13), pages 503-509,

### SEQUENCE LISTING

<110> FibroGen, Inc.
   olsen, David R.
   Polarek, James W.
   Yang, Chunlin
<120> IMPLANTABLE COLLAGEN COMPOSITIONS
<130> FP0305 PCT
<150> US 10/985,570
   <151> 2004-11-10
<160> 3
<170> PatentIn version 3.2
<210> 1
   <211> 1466
   <212> PRT
   <213> Homo sapiens
<400> 1
   Met Met Ser Phe Val Gln Lys Gly Ser Trp Leu Leu Leu Ala Leu Leu
   1 5 10 15
   His Pro Thr Ile Ile Leu Ala Gln Gln Glu Ala Val Glu Gly Gly Cys
   20 25 30
   Ser His Leu Gly Gln Ser Tyr Ala Asp Arg Asp Val Trp Lys Pro Glu
   35 40 45
   Pro Cys Gln Ile Cys Val Cys Asp Ser Gly Ser Val Leu Cys Asp Asp
   50 55 60
   Ile Ile Cys Asp Asp Gln Glu Leu Asp Cys Pro Asn Pro Glu Ile Pro
   65 70 75 80
   Phe Gly Glu Cys Cys Ala Val Cys Pro Gln Pro Pro Thr Ala Pro Thr
   85 90 95
   Arg Pro Pro Asn Gly Gln Gly Pro Gln Gly Pro Lys Gly Asp Pro Gly
   100 105 110
   Pro Pro Gly Ile Pro Gly Arg Asn Gly Asp Pro Gly Ile Pro Gly Gln
   115 120 125
   Pro Gly Ser Pro Gly Ser Pro Gly Pro Pro Gly Ile Cys Glu Ser Cys
   130 135 140
   Pro Thr Gly Pro Gln Asn Tyr Ser Pro Gln Tyr Asp Ser Tyr Asp Val
   145 150 155 160
   Lys Ser Gly Val Ala Val Gly Gly Leu Ala Gly Tyr Pro Gly Pro Ala
   165 170 175
   Gly Pro Pro Gly Pro Pro Gly Pro Pro Gly Thr Ser Gly His Pro Gly
   180 185 190
   Ser Pro Gly Ser Pro Gly Tyr Gln Gly Pro Pro Gly Glu Pro Gly Gln
   195 200 205
   Ala Gly Pro Ser Gly Pro Pro Gly Pro Pro Gly Ala Ile Gly Pro Ser
   210 215 220
   Gly Pro Ala Gly Lys Asp Gly Glu Ser Gly Arg Pro Gly Arg Pro Gly
   225 230 235 240
   Glu Arg Gly Leu Pro Gly Pro Pro Gly Ile Lys Gly Pro Ala Gly Ile
   245 250 255
   Pro Gly Phe Pro Gly Met Lys Gly His Arg Gly Phe Asp Gly Arg Asn
   260 265 270
   Gly Glu Lys Gly Glu Thr Gly Ala Pro Gly Leu Lys Gly Glu Asn Gly
   275 280 285
   Leu Pro Gly Glu Asn Gly Ala Pro Gly Pro Met Gly Pro Arg Gly Ala
   290 295 300
   Pro Gly Glu Arg Gly Arg Pro Gly Leu Pro Gly Ala Ala Gly Ala Arg
   305 310 315 320
   Gly Asn Asp Gly Ala Arg Gly Ser Asp Gly Gln Pro Gly Pro Pro Gly
   325 330 335
   Pro Pro Gly Thr Ala Gly Phe Pro Gly Ser Pro Gly Ala Lys Gly Glu
   340 345 350
   Val Gly Pro Ala Gly Ser Pro Gly Ser Asn Gly Ala Pro Gly Gln Arg
   355 360 365
   Gly Glu Pro Gly Pro Gln Gly His Ala Gly Ala Gln Gly Pro Pro Gly
   370 375 380
   Pro Pro Gly Ile Asn Gly Ser Pro Gly Gly Lys Gly Glu Met Gly Pro
   385 390 395 400
   Ala Gly Ile Pro Gly Ala Pro Gly Leu Met Gly Ala Arg Gly Pro Pro
   405 410 415
   Gly Pro Ala Gly Ala Asn Gly Ala Pro Gly Leu Arg Gly Gly Ala Gly
   420 425 430
   Glu Pro Gly Lys Asn Gly Ala Lys Gly Glu Pro Gly Pro Arg Gly Glu
   435 440 445
   Arg Gly Glu Ala Gly Ile Pro Gly Val Pro Gly Ala Lys Gly Glu Asp
       450                 455                 460
   Gly Lys Asp Gly Ser Pro Gly Glu Pro Gly Ala Asn Gly Leu Pro Gly
   465                 470                 475                 480
   Ala Ala Gly Glu Arg Gly Ala Pro Gly Phe Arg Gly Pro Ala Gly Pro
                   485                 490                 495
   Asn Gly Ile Pro Gly Glu Lys Gly Pro Ala Gly Glu Arg Gly Ala Pro
               500                 505                 510
   Gly Pro Ala Gly Pro Arg Gly Ala Ala Gly Glu Pro Gly Arg Asp Gly
           515                 520                 525
   Val Pro Gly Gly Pro Gly Met Arg Gly Met Pro Gly Ser Pro Gly Gly
       530                 535                 540
   Pro Gly Ser Asp Gly Lys Pro Gly Pro Pro Gly Ser Gln Gly Glu Ser
   545                 550                 555                 560
   Gly Arg Pro Gly Pro Pro Gly Pro Ser Gly Pro Arg Gly Gln Pro Gly
                   565                 570                 575
   Val Met Gly Phe Pro Gly Pro Lys Gly Asn Asp Gly Ala Pro Gly Lys
               580                 585                 590
   Asn Gly Glu Arg Gly Gly Pro Gly Gly Pro Gly Pro Gln Gly Pro Pro
           595                 600                 605
   Gly Lys Asn Gly Glu Thr Gly Pro Gln Gly Pro Pro Gly Pro Thr Gly
       610                 615                 620
   Pro Gly Gly Asp Lys Gly Asp Thr Gly Pro Pro Gly Pro Gln Gly Leu
   625                 630                 635                 640
   Gln Gly Leu Pro Gly Thr Gly Gly Pro Pro Gly Glu Asn Gly Lys Pro
                   645                 650                 655
   Gly Glu Pro Gly Pro Lys Gly Asp Ala Gly Ala Pro Gly Ala Pro Gly
               660                 665                 670
   Gly Lys Gly Asp Ala Gly Ala Pro Gly Glu Arg Gly Pro Pro Gly Leu
           675                 680                 685
   Ala Gly Ala Pro Gly Leu Arg Gly Gly Ala Gly Pro Pro Gly Pro Glu
       690                 695                 700
   Gly Gly Lys Gly Ala Ala Gly Pro Pro Gly Pro Pro Gly Ala Ala Gly
   705                 710                 715                 720
   Thr Pro Gly Leu Gln Gly Met Pro Gly Glu Arg Gly Gly Leu Gly Ser
   725 730 735
   Pro Gly Pro Lys Gly Asp Lys Gly Glu Pro Gly Gly Pro Gly Ala Asp
   740 745 750
   Gly Val Pro Gly Lys Asp Gly Pro Arg Gly Pro Thr Gly Pro Ile Gly
   755 760 765
   Pro Pro Gly Pro Ala Gly Gln Pro Gly Asp Lys Gly Glu Gly Gly Ala
   770 775 780
   Pro Gly Leu Pro Gly Ile Ala Gly Pro Arg Gly Ser Pro Gly Glu Arg
   785 790 795 800
   Gly Glu Thr Gly Pro Pro Gly Pro Ala Gly Phe Pro Gly Ala Pro Gly
   805 810 815
   Gln Asn Gly Glu Pro Gly Gly Lys Gly Glu Arg Gly Ala Pro Gly Glu
   820 825 830
   Lys Gly Glu Gly Gly Pro Pro Gly Val Ala Gly Pro Pro Gly Gly Ser
   835 840 845
   Gly Pro Ala Gly Pro Pro Gly Pro Gln Gly Val Lys Gly Glu Arg Gly
   850 855 860
   Ser Pro Gly Gly Pro Gly Ala Ala Gly Phe Pro Gly Ala Arg Gly Leu
   865 870 875 880
   Pro Gly Pro Pro Gly Ser Asn Gly Asn Pro Gly Pro Pro Gly Pro Ser
   885 890 895
   Gly Ser Pro Gly Lys Asp Gly Pro Pro Gly Pro Ala Gly Asn Thr Gly
   900 905 910
   Ala Pro Gly Ser Pro Gly Val Ser Gly Pro Lys Gly Asp Ala Gly Gln
   915 920 925
   Pro Gly Glu Lys Gly Ser Pro Gly Ala Gln Gly Pro Pro Gly Ala Pro
   930 935 940
   Gly Pro Leu Gly Ile Ala Gly Ile Thr Gly Ala Arg Gly Leu Ala Gly
   945 950 955 960
   Pro Pro Gly Met Pro Gly Pro Arg Gly Ser Pro Gly Pro Gln Gly Val
   965 970 975
   Lys Gly Glu Ser Gly Lys Pro Gly Ala Asn Gly Leu Ser Gly Glu Arg
   980 985 990
   Gly Pro Pro Gly Pro Gln Gly Leu Pro Gly Leu Ala Gly Thr Ala Gly
   995 1000 1005
   Glu Pro Gly Arg Asp Gly Asn Pro Gly Ser Asp Gly Leu Pro Gly
   1010 1015 1020
   Arg Asp Gly Ser Pro Gly Gly Lys Gly Asp Arg Gly Glu Asn Gly
   1025 1030 1035
   Ser Pro Gly Ala Pro Gly Ala Pro Gly His Pro Gly Pro Pro Gly
   1040 1045 1050
   Pro Val Gly Pro Ala Gly Lys Ser Gly Asp Arg Gly Glu Ser Gly
   1055 1060 1065
   Pro Ala Gly Pro Ala Gly Ala Pro Gly Pro Ala Gly Ser Arg Gly
   1070 1075 1080
   Ala Pro Gly Pro Gln Gly Pro Arg Gly Asp Lys Gly Glu Thr Gly
   1085 1090 1095
   Glu Arg Gly Ala Ala Gly Ile Lys Gly His Arg Gly Phe Pro Gly
   1100 1105 1110
   Asn Pro Gly Ala Pro Gly Ser Pro Gly Pro Ala Gly Gln Gln Gly
   1115 1120 1125
   Ala Ile Gly Ser Pro Gly Pro Ala Gly Pro Arg Gly Pro Val Gly
   1130 1135 1140
   Pro Ser Gly Pro Pro Gly Lys Asp Gly Thr Ser Gly His Pro Gly
   1145 1150 1155
   Pro Ile Gly Pro Pro Gly Pro Arg Gly Asn Arg Gly Glu Arg Gly
   1160 1165 1170
   Ser Glu Gly Ser Pro Gly His Pro Gly Gln Pro Gly Pro Pro Gly
   1175 1180 1185
   Pro Pro Gly Ala Pro Gly Pro Cys Cys Gly Gly Val Gly Ala Ala
   1190 1195 1200
   Ala Ile Ala Gly Ile Gly Gly Glu Lys Ala Gly Gly Phe Ala Pro
   1205 1210 1215
   Tyr Tyr Gly Asp Glu Pro Met Asp Phe Lys Ile Asn Thr Asp Glu
   1220 1225 1230
   Ile Met Thr Ser Leu Lys Ser Val Asn Gly Gln Ile Glu Ser Leu
   1235 1240 1245
   Ile Ser Pro Asp Gly Ser Arg Lys Asn Pro Ala Arg Asn Cys Arg
   1250 1255 1260
   Asp Leu Lys Phe Cys His Pro Glu Leu Lys Ser Gly Glu Tyr Trp
   1265 1270 1275
   Val Asp Pro Asn Gln Gly Cys Lys Leu Asp Ala Ile Lys Val Phe
   1280 1285 1290
   Cys Asn Met Glu Thr Gly Glu Thr Cys Ile Ser Ala Asn Pro Leu
   1295 1300 1305
   Asn Val Pro Arg Lys His Trp Trp Thr Asp Ser Ser Ala Glu Lys
   1310 1315 1320
   Lys His Val Trp Phe Gly Glu Ser Met Asp Gly Gly Phe Gln Phe
   1325 1330 1335
   Ser Tyr Gly Asn Pro Glu Leu Pro Glu Asp Val Leu Asp Val Gln
   1340 1345 1350
   Leu Ala Phe Leu Arg Leu Leu Ser Ser Arg Ala Ser Gln Asn Ile
   1355 1360 1365
   Thr Tyr His Cys Lys Asn Ser Ile Ala Tyr Met Asp Gln Ala Ser
   1370 1375 1380
   Gly Asn Val Lys Lys Ala Leu Lys Leu Met Gly Ser Asn Glu Gly
   1385 1390 1395
   Glu Phe Lys Ala Glu Gly Asn Ser Lys Phe Thr Tyr Thr Val Leu
   1400 1405 1410
   Glu Asp Gly Cys Thr Lys His Thr Gly Glu Trp Ser Lys Thr Val
   1415 1420 1425
   Phe Glu Tyr Arg Thr Arg Lys Ala Val Arg Leu Pro Ile Val Asp
   1430 1435 1440
   Ile Ala Pro Tyr Asp Ile Gly Gly Pro Asp Gln Glu Phe Gly Val
   1445 1450 1455
   Asp Val Gly Pro Val Cys Phe Leu
   1460 1465
<210> 2
   <211> 1464
   <212> PRT
   <213> Homo sapiens
<400> 2
   Met Phe Ser Phe Val Asp Leu Arg Leu Leu Leu Leu Leu Ala Ala Thr
   1               5                   10                  15
   Ala Leu Leu Thr His Gly Gln Glu Glu Gly Gln Val Glu Gly Gln Asp
               20                  25                  30
   Glu Asp Ile Pro Pro Ile Thr Cys Val Gln Asn Gly Leu Arg Tyr His
           35                  40                  45
   Asp Arg Asp Val Trp Lys Pro Glu Pro Cys Arg Ile Cys Val Cys Asp
       50                  55                  60
   Asn Gly Lys Val Leu Cys Asp Asp Val Ile Cys Asp Glu Thr Lys Asn
   65                  70                  75                  80
   Cys Pro Gly Ala Glu Val Pro Glu Gly Glu Cys Cys Pro Val Cys Pro
                   85                  90                  95
   Asp Gly Ser Glu Ser Pro Thr Asp Gln Glu Thr Thr Gly Val Glu Gly
               100                 105                 110
   Pro Lys Gly Asp Thr Gly Pro Arg Gly Pro Arg Gly Pro Ala Gly Pro
           115                 120                 125
   Pro Gly Arg Asp Gly Ile Pro Gly Gln Pro Gly Leu Pro Gly Pro Pro
       130                 135                 140
   Gly Pro Pro Gly Pro Pro Gly Pro Pro Gly Leu Gly Gly Asn Phe Ala
   145                 150                 155                 160
   Pro Gln Leu Ser Tyr Gly Tyr Asp Glu Lys Ser Thr Gly Gly Ile Ser
                   165                 170                 175
   Val Pro Gly Pro Met Gly Pro Ser Gly Pro Arg Gly Leu Pro Gly Pro
               180                 185                 190
   Pro Gly Ala Pro Gly Pro Gln Gly Phe Gln Gly Pro Pro Gly Glu Pro
           195                 200                 205
   Gly Glu Pro Gly Ala Ser Gly Pro Met Gly Pro Arg Gly Pro Pro Gly
       210                 215                 220
   Pro Pro Gly Lys Asn Gly Asp Asp Gly Glu Ala Gly Lys Pro Gly Arg
   225                 230                 235                 240
   Pro Gly Glu Arg Gly Pro Pro Gly Pro Gln Gly Ala Arg Gly Leu Pro
                   245                 250                 255
   Gly Thr Ala Gly Leu Pro Gly Met Lys Gly His Arg Gly Phe Ser Gly
               260                 265                 270
   Leu Asp Gly Ala Lys Gly Asp Ala Gly Pro Ala Gly Pro Lys Gly Glu
   275 280 285
   Pro Gly Ser Pro Gly Glu Asn Gly Ala Pro Gly Gln Met Gly Pro Arg
   290 295 300
   Gly Leu Pro Gly Glu Arg Gly Arg Pro Gly Ala Pro Gly Pro Ala Gly
   305 310 315 320
   Ala Arg Gly Asn Asp Gly Ala Thr Gly Ala Ala Gly Pro Pro Gly Pro
   325 330 335
   Thr Gly Pro Ala Gly Pro Pro Gly Phe Pro Gly Ala Val Gly Ala Lys
   340 345 350
   Gly Glu Ala Gly Pro Gln Gly Pro Arg Gly Ser Glu Gly Pro Gln Gly
   355 360 365
   Val Arg Gly Glu Pro Gly Pro Pro Gly Pro Ala Gly Ala Ala Gly Pro
   370 375 380
   Ala Gly Asn Pro Gly Ala Asp Gly Gln Pro Gly Ala Lys Gly Ala Asn
   385 390 395 400
   Gly Ala Pro Gly Ile Ala Gly Ala Pro Gly Phe Pro Gly Ala Arg Gly
   405 410 415
   Pro Ser Gly Pro Gln Gly Pro Gly Gly Pro Pro Gly Pro Lys Gly Asn
   420 425 430
   Ser Gly Glu Pro Gly Ala Pro Gly Ser Lys Gly Asp Thr Gly Ala Lys
   435 440 445
   Gly Glu Pro Gly Pro Val Gly Val Gln Gly Pro Pro Gly Pro Ala Gly
   450 455 460
   Glu Glu Gly Lys Arg Gly Ala Arg Gly Glu Pro Gly Pro Thr Gly Leu
   465 470 475 480
   Pro Gly Pro Pro Gly Glu Arg Gly Gly Pro Gly Ser Arg Gly Phe Pro
   485 490 495
   Gly Ala Asp Gly Val Ala Gly Pro Lys Gly Pro Ala Gly Glu Arg Gly
   500 505 510
   Ser Pro Gly Pro Ala Gly Pro Lys Gly Ser Pro Gly Glu Ala Gly Arg
   515 520 525
   Pro Gly Glu Ala Gly Leu Pro Gly Ala Lys Gly Leu Thr Gly Ser Pro
   530 535 540
   Gly Ser Pro Gly Pro Asp Gly Lys Thr Gly Pro Pro Gly Pro Ala Gly
   545 550 555 560
   Gln Asp Gly Arg Pro Gly Pro Pro Gly Pro Pro Gly Ala Arg Gly Gln
   565 570 575
   Ala Gly Val Met Gly Phe Pro Gly Pro Lys Gly Ala Ala Gly Glu Pro
   580 585 590
   Gly Lys Ala Gly Glu Arg Gly Val Pro Gly Pro Pro Gly Ala Val Gly
   595 600 605
   Pro Ala Gly Lys Asp Gly Glu Ala Gly Ala Gln Gly Pro Pro Gly Pro
   610 615 620
   Ala Gly Pro Ala Gly Glu Arg Gly Glu Gln Gly Pro Ala Gly Ser Pro
   625 630 635 640
   Gly Phe Gln Gly Leu Pro Gly Pro Ala Gly Pro Pro Gly Glu Ala Gly
   645 650 655
   Lys Pro Gly Glu Gln Gly Val Pro Gly Asp Leu Gly Ala Pro Gly Pro
   660 665 670
   Ser Gly Ala Arg Gly Glu Arg Gly Phe Pro Gly Glu Arg Gly Val Gln
   675 680 685
   Gly Pro Pro Gly Pro Ala Gly Pro Arg Gly Ala Asn Gly Ala Pro Gly
   690 695 700
   Asn Asp Gly Ala Lys Gly Asp Ala Gly Ala Pro Gly Ala Pro Gly Ser
   705 710 715 720
   Gln Gly Ala Pro Gly Leu Gln Gly Met Pro Gly Glu Arg Gly Ala Ala
   725 730 735
   Gly Leu Pro Gly Pro Lys Gly Asp Arg Gly Asp Ala Gly Pro Lys Gly
   740 745 750
   Ala Asp Gly Ser Pro Gly Lys Asp Gly Val Arg Gly Leu Thr Gly Pro
   755 760 765
   Ile Gly Pro Pro Gly Pro Ala Gly Ala Pro Gly Asp Lys Gly Glu Ser
   770 775 780
   Gly Pro Ser Gly Pro Ala Gly Pro Thr Gly Ala Arg Gly Ala Pro Gly
   785 790 795 800
   Asp Arg Gly Glu Pro Gly Pro Pro Gly Pro Ala Gly Phe Ala Gly Pro
   805 810 815
   Pro Gly Ala Asp Gly Gln Pro Gly Ala Lys Gly Glu Pro Gly Asp Ala
   820 825 830
   Gly Ala Lys Gly Asp Ala Gly Pro Pro Gly Pro Ala Gly Pro Ala Gly
   835 840 845
   Pro Pro Gly Pro Ile Gly Asn Val Gly Ala Pro Gly Ala Lys Gly Ala
   850 855 860
   Arg Gly Ser Ala Gly Pro Pro Gly Ala Thr Gly Phe Pro Gly Ala Ala
   865 870 875 880
   Gly Arg Val Gly Pro Pro Gly Pro Ser Gly Asn Ala Gly Pro Pro Gly
   885 890 895
   Pro Pro Gly Pro Ala Gly Lys Glu Gly Gly Lys Gly Pro Arg Gly Glu
   900 905 910
   Thr Gly Pro Ala Gly Arg Pro Gly Glu Val Gly Pro Pro Gly Pro Pro
   915 920 925
   Gly Pro Ala Gly Glu Lys Gly Ser Pro Gly Ala Asp Gly Pro Ala Gly
   930 935 940
   Ala Pro Gly Thr Pro Gly Pro Gln Gly Ile Ala Gly Gln Arg Gly Val
   945 950 955 960
   Val Gly Leu Pro Gly Gln Arg Gly Glu Arg Gly Phe Pro Gly Leu Pro
   965 970 975
   Gly Pro Ser Gly Glu Pro Gly Lys Gln Gly Pro Ser Gly Ala Ser Gly
   980 985 990
   Glu Arg Gly Pro Pro Gly Pro Met Gly Pro Pro Gly Leu Ala Gly Pro
   995 1000 1005
   Pro Gly Glu Ser Gly Arg Glu Gly Ala Pro Gly Ala Glu Gly Ser
   1010 1015 1020
   Pro Gly Arg Asp Gly Ser Pro Gly Ala Lys Gly Asp Arg Gly Glu
   1025 1030 1035
   Thr Gly Pro Ala Gly Pro Pro Gly Ala Pro Gly Ala Pro Gly Ala
   1040 1045 1050
   Pro Gly Pro Val Gly Pro Ala Gly Lys Ser Gly Asp Arg Gly Glu
   1055 1060 1065
   Thr Gly Pro Ala Gly Pro Ala Gly Pro Val Gly Pro Ala Gly Ala
   1070 1075 1080
   Arg Gly Pro Ala Gly Pro Gln Gly Pro Arg Gly Asp Lys Gly Glu
   1085 1090 1095
   Thr Gly Glu Gln Gly Asp Arg Gly Ile Lys Gly His Arg Gly Phe
   1100 1105 1110
   Ser Gly Leu Gln Gly Pro Pro Gly Pro Pro Gly Ser Pro Gly Glu
   1115 1120 1125
   Gln Gly Pro Ser Gly Ala Ser Gly Pro Ala Gly Pro Arg Gly Pro
   1130 1135 1140
   Pro Gly Ser Ala Gly Ala Pro Gly Lys Asp Gly Leu Asn Gly Leu
   1145 1150 1155
   Pro Gly Pro Ile Gly Pro Pro Gly Pro Arg Gly Arg Thr Gly Asp
   1160 1165 1170
   Ala Gly Pro Val Gly Pro Pro Gly Pro Pro Gly Pro Pro Gly Pro
   1175 1180 1185
   Pro Gly Pro Pro Ser Ala Gly Phe Asp Phe Ser Phe Leu Pro Gln
   1190 1195 1200
   Pro Pro Gln Glu Lys Ala His Asp Gly Gly Arg Tyr Tyr Arg Ala
   1205 1210 1215
   Asp Asp Ala Asn Val Val Arg Asp Arg Asp Leu Glu Val Asp Thr
   1220 1225 1230
   Thr Leu Lys Ser Leu Ser Gln Gln Ile Glu Asn Ile Arg Ser Pro
   1235 1240 1245
   Glu Gly Ser Arg Lys Asn Pro Ala Arg Thr Cys Arg Asp Leu Lys
   1250 1255 1260
   Met Cys His Ser Asp Trp Lys Ser Gly Glu Tyr Trp Ile Asp Pro
   1265 1270 1275
   Asn Gln Gly Cys Asn Leu Asp Ala Ile Lys Val Phe Cys Asn Met
   1280 1285 1290
   Glu Thr Gly Glu Thr Cys Val Tyr Pro Thr Gln Pro Ser Val Ala
   1295 1300 1305
   Gln Lys Asn Trp Tyr Ile Ser Lys Asn Pro Lys Asp Lys Arg His
   1310 1315 1320
   Val Trp Phe Gly Glu Ser Met Thr Asp Gly Phe Gln Phe Glu Tyr
   1325 1330 1335
   Gly Gly Gln Gly Ser Asp Pro Ala Asp Val Ala Ile Gln Leu Thr
   1340 1345 1350
   Phe Leu Arg Leu Met Ser Thr Glu Ala Ser Gln Asn Ile Thr Tyr
   1355 1360 1365
   His Cys Lys Asn Ser Val Ala Tyr Met Asp Gln Gln Thr Gly Asn
   1370 1375 1380
   Leu Lys Lys Ala Leu Leu Leu Lys Gly Ser Asn Glu Ile Glu Ile
   1385 1390 1395
   Arg Ala Glu Gly Asn Ser Arg Phe Thr Tyr Ser Val Thr Val Asp
   1400 1405 1410
   Gly Cys Thr Ser His Thr Gly Ala Trp Gly Lys Thr Val Ile Glu
   1415 1420 1425
   Tyr Lys Thr Thr Lys Thr Ser Arg Leu Pro Ile Ile Asp Val Ala
   1430 1435 1440
   Pro Leu Asp Val Gly Ala Pro Asp Gln Glu Phe Gly Phe Asp Val
   1445 1450 1455
   Gly Pro Val Cys Phe Leu
   1460
<210> 3
   <211> 1366
   <212> PRT
   <213> Homo sapiens
<400> 3
   Met Leu Ser Phe Val Asp Thr Arg Thr Leu Leu Leu Leu Ala Val Thr
   1 5 10 15
   Leu Cys Leu Ala Thr Cys Gln Ser Leu Gln Glu Glu Thr Val Arg Lys
   20 25 30
   Gly Pro Ala Gly Asp Arg Gly Pro Arg Gly Glu Arg Gly Pro Pro Gly
   35 40 45
   Pro Pro Gly Arg Asp Gly Glu Asp Gly Pro Thr Gly Pro Pro Gly Pro
   50 55 60
   Pro Gly Pro Pro Gly Pro Pro Gly Leu Gly Gly Asn Phe Ala Ala Gln
   65 70 75 80
   Tyr Asp Gly Lys Gly Val Gly Leu Gly Pro Gly Pro Met Gly Leu Met
   85 90 95
   Gly Pro Arg Gly Pro Pro Gly Ala Ala Gly Ala Pro Gly Pro Gln Gly
   100 105 110
   Phe Gln Gly Pro Ala Gly Glu Pro Gly Glu Pro Gly Gln Thr Gly Pro
   115 120 125
   Ala Gly Ala Arg Gly Pro Ala Gly Pro Pro Gly Lys Ala Gly Glu Asp
   130 135 140
   Gly His Pro Gly Lys Pro Gly Arg Pro Gly Glu Arg Gly Val Val Gly
   145 150 155 160
   Pro Gln Gly Ala Arg Gly Phe Pro Gly Thr Pro Gly Leu Pro Gly Phe
   165 170 175
   Lys Gly Ile Arg Gly His Asn Gly Leu Asp Gly Leu Lys Gly Gln Pro
   180 185 190
   Gly Ala Pro Gly Val Lys Gly Glu Pro Gly Ala Pro Gly Glu Asn Gly
   195 200 205
   Thr Pro Gly Gln Thr Gly Ala Arg Gly Leu Pro Gly Glu Arg Gly Arg
   210 215 220
   Val Gly Ala Pro Gly Pro Ala Gly Ala Arg Gly Ser Asp Gly Ser Val
   225 230 235 240
   Gly Pro Val Gly Pro Ala Gly Pro Asn Gly Ser Ala Gly Pro Pro Gly
   245 250 255
   Phe Pro Gly Ala Pro Gly Pro Lys Gly Glu Ile Gly Ala Val Gly Asn
   260 265 270
   Ala Gly Pro Thr Gly Pro Ala Gly Pro Arg Gly Glu Val Gly Leu Pro
   275 280 285
   Gly Leu Ser Gly Pro Val Gly Pro Pro Gly Asn Pro Gly Ala Asn Gly
   290 295 300
   Leu Thr Gly Ala Lys Gly Ala Ala Gly Leu Pro Gly Val Ala Gly Ala
   305 310 315 320
   Pro Gly Leu Pro Gly Pro Arg Gly Ile Pro Gly Pro Pro Gly Ala Ala
   325 330 335
   Gly Thr Thr Gly Ala Arg Gly Leu Val Gly Glu Pro Gly Pro Ala Gly
   340 345 350
   Ser Lys Gly Glu Ser Gly Asn Lys Gly Glu Pro Gly Ser Ala Gly Pro
   355 360 365
   Gln Gly Pro Pro Gly Pro Ser Gly Glu Glu Gly Lys Arg Gly Pro Asn
                   370                 375                 380
   Gly Glu Ala Gly Ser Ala Gly Pro Pro Gly Pro Pro Gly Leu Arg Gly
   385                 390                 395                 400
   Ser Pro Gly Ser Arg Gly Leu Pro Gly Ala Asp Gly Arg Ala Gly Val
                   405                 410                 415
   Met Gly Pro Pro Gly Ser Arg Gly Ala Ser Gly Pro Ala Gly Val Arg
               420                 425                 430
   Gly Pro Asn Gly Asp Ala Gly Arg Pro Gly Glu Pro Gly Leu Met Gly
           435                 440                 445
   Pro Arg Gly Leu Pro Gly Ser Pro Gly Asn Ile Gly Pro Ala Gly Lys
       450                 455                 460
   Glu Gly Pro Val Gly Leu Pro Gly Ile Asp Gly Arg Pro Gly Pro Ile
   465                 470                 475                 480
   Gly Pro Val Gly Ala Arg Gly Glu Pro Gly Asn Ile Gly Phe Pro Gly
                   485                 490                 495
   Pro Lys Gly Pro Thr Gly Asp Pro Gly Lys Asn Gly Asp Lys Gly His
               500                 505                 510
   Ala Gly Leu Ala Gly Ala Arg Gly Ala Pro Gly Pro Asp Gly Asn Asn
           515                 520                 525
   Gly Ala Gln Gly Pro Pro Gly Pro Gln Gly Val Gln Gly Gly Lys Gly
       530                 535                 540
   Glu Gln Gly Pro Ala Gly Pro Pro Gly Phe Gln Gly Leu Pro Gly Pro
   545                 550                 555                 560
   Ser Gly Pro Ala Gly Glu Val Gly Lys Pro Gly Glu Arg Gly Leu His
                   565                 570                 575
   Gly Glu Phe Gly Leu Pro Gly Pro Ala Gly Pro Arg Gly Glu Arg Gly
               580                 585                 590
   Pro Pro Gly Glu Ser Gly Ala Ala Gly Pro Thr Gly Pro Ile Gly Ser
           595                 600                 605
   Arg Gly Pro Ser Gly Pro Pro Gly Pro Asp Gly Asn Lys Gly Glu Pro
       610                 615                 620
   Gly Val Val Gly Ala Val Gly Thr Ala Gly Pro Ser Gly Pro Ser Gly
   625                 630                 635                 640
   Leu Pro Gly Glu Arg Gly Ala Ala Gly Ile Pro Gly Gly Lys Gly Glu
   645 650 655
   Lys Gly Glu Pro Gly Leu Arg Gly Glu Ile Gly Asn Pro Gly Arg Asp
   660 665 670
   Gly Ala Arg Gly Ala His Gly Ala Val Gly Ala Pro Gly Pro Ala Gly
   675 680 685
   Ala Thr Gly Asp Arg Gly Glu Ala Gly Ala Ala Gly Pro Ala Gly Pro
   690 695 700
   Ala Gly Pro Arg Gly Ser Pro Gly Glu Arg Gly Glu Val Gly Pro Ala
   705 710 715 720
   Gly Pro Asn Gly Phe Ala Gly Pro Ala Gly Ala Ala Gly Gln Pro Gly
   725 730 735
   Ala Lys Gly Glu Arg Gly Gly Lys Gly Pro Lys Gly Glu Asn Gly Val
   740 745 750
   Val Gly Pro Thr Gly Pro Val Gly Ala Ala Gly Pro Ala Gly Pro Asn
   755 760 765
   Gly Pro Pro Gly Pro Ala Gly Ser Arg Gly Asp Gly Gly Pro Pro Gly
   770 775 780
   Met Thr Gly Phe Pro Gly Ala Ala Gly Arg Thr Gly Pro Pro Gly Pro
   785 790 795 800
   Ser Gly Ile Ser Gly Pro Pro Gly Pro Pro Gly Pro Ala Gly Lys Glu
   805 810 815
   Gly Leu Arg Gly Pro Arg Gly Asp Gln Gly Pro Val Gly Arg Thr Gly
   820 825 830
   Glu Val Gly Ala Val Gly Pro Pro Gly Phe Ala Gly Glu Lys Gly Pro
   835 840 845
   Ser Gly Glu Ala Gly Thr Ala Gly Pro Pro Gly Thr Pro Gly Pro Gln
   850 855 860
   Gly Leu Leu Gly Ala Pro Gly Ile Leu Gly Leu Pro Gly Ser Arg Gly
   865 870 875 880
   Glu Arg Gly Leu Pro Gly Val Ala Gly Ala Val Gly Glu Pro Gly Pro
   885 890 895
   Leu Gly Ile Ala Gly Pro Pro Gly Ala Arg Gly Pro Pro Gly Ala Val
   900 905 910
   Gly Ser Pro Gly Val Asn Gly Ala Pro Gly Glu Ala Gly Arg Asp Gly
   915 920 925
   Asn Pro Gly Asn Asp Gly Pro Pro Gly Arg Asp Gly Gln Pro Gly His
   930 935 940
   Lys Gly Glu Arg Gly Tyr Pro Gly Asn Ile Gly Pro Val Gly Ala Ala
   945 950 955 960
   Gly Ala Pro Gly Pro His Gly Pro Val Gly Pro Ala Gly Lys His Gly
   965 970 975
   Asn Arg Gly Glu Thr Gly Pro Ser Gly Pro Val Gly Pro Ala Gly Ala
   980 985 990
   Val Gly Pro Arg Gly Pro Ser Gly Pro Gln Gly Ile Arg Gly Asp Lys
   995 1000 1005
   Gly Glu Pro Gly Glu Lys Gly Pro Arg Gly Leu Pro Gly Phe Lys
   1010 1015 1020
   Gly His Asn Gly Leu Gln Gly Leu Pro Gly Ile Ala Gly His His
   1025 1030 1035
   Gly Asp Gln Gly Ala Pro Gly Ser Val Gly Pro Ala Gly Pro Arg
   1040 1045 1050
   Gly Pro Ala Gly Pro Ser Gly Pro Ala Gly Lys Asp Gly Arg Thr
   1055 1060 1065
   Gly His Pro Gly Thr Val Gly Pro Ala Gly Ile Arg Gly Pro Gln
   1070 1075 1080
   Gly His Gln Gly Pro Ala Gly Pro Pro Gly Pro Pro Gly Pro Pro
   1085 1090 1095
   Gly Pro Pro Gly Val Ser Gly Gly Gly Tyr Asp Phe Gly Tyr Asp
   1100 1105 1110
   Gly Asp Phe Tyr Arg Ala Asp Gln Pro Arg Ser Ala Pro Ser Leu
   1115 1120 1125
   Arg Pro Lys Asp Tyr Glu Val Asp Ala Thr Leu Lys Ser Leu Asn
   1130 1135 1140
   Asn Gln Ile Glu Thr Leu Leu Thr Pro Glu Gly Ser Arg Lys Asn
   1145 1150 1155
   Pro Ala Arg Thr Cys Arg Asp Leu Arg Leu Ser His Pro Glu Trp
   1160 1165 1170
   Ser Ser Gly Tyr Tyr Trp Ile Asp Pro Asn Gln Gly Cys Thr Met
   1175 1180 1185
   Glu Ala Ile Lys Val Tyr Cys Asp Phe Pro Thr Gly Glu Thr Cys
   1190 1195 1200
   Ile Arg Ala Gln Pro Glu Asn Ile Pro Ala Lys Asn Trp Tyr Arg
   1205 1210 1215
   Ser Ser Lys Asp Lys Lys His Val Trp Leu Gly Glu Thr Ile Asn
   1220 1225 1230
   Ala Gly Ser Gln Phe Glu Tyr Asn Val Glu Gly Val Thr Ser Lys
   1235 1240 1245
   Glu Met Ala Thr Gln Leu Ala Phe Met Arg Leu Leu Ala Asn Tyr
   1250 1255 1260
   Ala Ser Gln Asn Ile Thr Tyr His Cys Lys Asn Ser Ile Ala Tyr
   1265 1270 1275
   Met Asp Glu Glu Thr Gly Asn Leu Lys Lys Ala Val Ile Leu Gln
   1280 1285 1290
   Gly Ser Asn Asp Val Glu Leu Val Ala Glu Gly Asn Ser Arg Phe
   1295 1300 1305
   Thr Tyr Thr Val Leu Val Asp Gly Cys Ser Lys Lys Thr Asn Glu
   1310 1315 1320
   Trp Gly Lys Thr Ile Ile Glu Tyr Lys Thr Asn Lys Pro Ser Arg
   1325 1330 1335
   Leu Pro Phe Leu Asp Ile Ala Pro Leu Asp Ile Gly Gly Ala Asp
   1340 1345 1350
   His Glu Phe Phe Val Asp Ile Gly Pro Val Cys Phe Lys
   1355 1360 1365

## Description

### FIELD OF THE INVENTION

The present invention relates to collagen compositions suitable for use in various tissue augmentation procedures.

### BACKGROUND OF THE INVENTION

Collagen has been widely used in numerous medical and cosmetic applications, including soft tissue augmentation procedures. Collagen used in these applications is endogenous collagen typically extracted from natural sources, such as animal, particularly bovine and porcine, or human tissues. Bovine collagen, in particular, has gained widespread use as an implant material for soft tissue augmentation.

Persistence is a critical feature of material to be implanted and used in various tissue augmentation procedures. The collagen that is the main bulking and structural material in most tissue augmentation products is susceptible upon implantation to degradation by host collagenases, and thus tends to degrade over a fairly short period of time. Lack of persistence due to degradation or resorption of the material can impair or compromise desired results, and retreatment can be required. Therefore, patients in need of or desiring continuous treatment or effects of longer duration must receive additional injections as early as the second or third month post-injection. (See, e.g., L. Baumann and E. Weisberg, "Soft Tissue Augmentation," Chapter 19, in Cosmetic Dermatology Principles and Practice, pp. 155-172, 2002, McGraw Hill, NY, NY, which discusses the impermanence of commercially available dermal filler products, and lists the duration of, e.g., ZYPLAST and ZYDERM bovine collagen dermal fillers (Inamed Corp., Santa Barbara, CA) at 2-4 months.) In the case of voluntary cosmetic procedures, the inconvenience, cost, and discomfort associated with a particular procedure can discourage prospective patients from obtaining retreatment, or from choosing to undergo a procedure in the first place, knowing the desired effects will be short-lived.

EP-A-0713707 describes an in situ crosslinkable injectable fibrillar collagen, i.e. type I and type III collagen composition for tissue augmentation.

US-B1-6509031 refers to a method of sealing wound comprising collagen and a crosslinking agent wherein the collagen is selected from the group of Type I collagen, Type II collagen, Type III collagen and Type IV collagen.

WO2004/078120 describes collagen compositions suitable for medical applications such as scaffolds, stents, tissue grafts, tissue and vascular sealants.

Various modifications directed to overcoming the lack of persistence/short duration of tissue-derived collagen bulking agents include both efforts to alter the physical properties of extracted collagen, e.g., by chemical crosslinking of the collagen source material (use of chemical agents to impose additional crosslinks on extracted collagen) (see, e.g., Narins et al. (2003) Dermatol. Surg. 29:588-595), and by increasing the concentration of collagen in the augmentation or bulking product (see, e.g., U.S. Patent Number 5,428,024). However, these formulations result in collagen with compromised/reduced manipulability, extrudability, and intrudability. Therefore, there is a need in the art for a collagen material having increased persistence for use in various medical and cosmetic applications. There is a further need for a collagen material that has increased persistence, with no concomitant reduction in or compromise of manipulability, extrudability, and intrudability.

The present invention addresses these needs by providing implantable collagen compositions having improved persistence, e.g., collagen compositions with persistence greater than that of commercially available collagen-based tissue augmentation products. Implantable collagen materials with minimal or no variability are also provided. The present invention further provides collagen compositions having improved manipulability, extrudability, and intrudability.

### SUMMARY OF THE INVENTION

The present invention relates to various implantable compositions comprising collagen consisting of type III collagen and offering enhanced persistence, improved manipulability and ease on handling, and uniformity and reproducibility. These compositions are suitable for use in various medical and cosmetic procedures, and, in particular, in tissue augmentation procedures. Methods for producing and for using these implantable compositions are provided, as are kits comprising them.

For purposes of the present invention, an implantable composition is a composition that can be administered to a tissue to support, augment, strengthen, or enhance the tissue. In particular, it is contemplated that an implantable composition is one suitable for *in vivo* administration and for use as an implant or implantable device. The implantable compositions of the present invention include injectable compositions, e.g., compositions that can be administered by injection. It is further contemplated that the implantable compositions of the present invention are suitable for subcutaneous, intradermal, or subdermal administration, and are suitable for use in soft tissue augmentation and hard tissue augmentation.

The present invention provides an implantable composition comprising collagen, wherein the collagen consists of type III collagen. In a particular aspect, the type III collagen is human type III collagen. In another aspect, the type III collagen is synthetic type III collagen. In yet another aspect, the type III collagen is free of any endogenous crosslinks. In a further aspect, the type III collagen is free of any intramolecular and intermolecular crosslinks. In certain aspects, the type III collagen comprises the amino acid sequence of SEQ ID NO:1 or collagenous, e.g., triple helical, fragments thereof. In other aspects, the type III collagen comprises amino acid residue 149 to amino acid residue 1221 of the amino acid sequence of SEQ ID NO:1 or collagenous, e.g., triple helical, fragments thereof. In yet other aspects, the type III collagen comprises amino acid residue 168 to amino acid residue 1196 of the amino acid sequence of SEQ ID NO: 1 or collagenous, e.g., triple helical, fragments thereof.

In various aspects, the implantable composition comprising collagen, wherein the collagen consists of type III collagen, is an injectable composition, e.g., is suitable for delivery by injection. It is contemplated in some aspects that the delivery is subcutaneous, intradermal, or subdermal. In particular aspects, the implantable composition is selected from the group consisting of a dermal filler, a bone-void filler, and a dental implant. The invention further encompasses aspects in which the implantable composition is suitable for use in tissue augmentation, and particular aspects in which the tissue augmentation is soft tissue augmentation and in which the tissue augmentation is hard tissue augmentation.

An implantable composition comprising collagen, wherein the collagen is free of intermolecular and intramolecular crosslinks, is contemplated herein. The collagen consists of type III collagen. In one aspect, the collagen is fibrillar collagen, and is selected from the group consisting of type III collagen. In a preferred aspect, the collagen is human type III collagen. It is contemplated that, in various aspects, the collagen is synthetic collagen. In a particular aspects, the collagen comprises the amino acid sequence of SEQ ID NO:1 or collagenous, e.g., triple helical, fragments thereof. In other aspects, the type III collagen comprises amino acid residue 149 to amino acid residue 1221 of the amino acid sequence of SEQ ID NO: 1 or collagenous, e.g., triple helical, fragments thereof. In yet other aspects, the type III collagen comprises amino acid residue 168 to amino acid residue 1196 of the amino acid sequence of SEQ ID NO:1 or collagenous, e.g., triple helical, fragments thereof.

The invention further provides an implantable composition comprising collagen free of intramolecular and intermolecular crosslinks, wherein the implantable composition is selected from the group consisting of a dermal filler, a bone-void filler, and a dental implant. The implantable composition is for use in tissue augmentation. Embodiments in which the tissue augmentation is soft tissue augmentation and in which the tissue augmentation is hard tissue augmentation are specifically contemplated.

Implantable compositions comprising collagen, wherein the collagen is free of endogenous crosslinks, are contemplated by the present invention. Collagens free of endogenous crosslinks may include but are not limited to collagens that, while free of endogenous crosslinks, have been exposed subsequent to endogenous production to crosslinking agents (chemical crosslinking agents, e.g., gluteraldehyde, etc.) or to crosslinking conditions (e.g., heat, radiation, etc.), such that the collagen does contain non-endogenously imposed crosslinks.

In one embodiment, the collagen free of endogenous crosslinks is fibrillar collagen. The collagen is selected from the group consisting of type III collagen. In other embodiments, the collagen is free of endogenous crosslinks. In a particular embodiment, the collagen is synthetic collagen. In one embodiment, the collagen is human collagen. In another embodiment, the collagen comprises the amino acid sequence of SEQ ID NO:1 or collagenous, e.g., triple helical, fragments thereof. In other embodiments, the type III collagen comprises amino acid residue 149 to amino acid residue 1221 of the amino acid sequence of SEQ ID NO:1 or collagenous, e.g., triple helical, fragments thereof. In yet other embodiments, the type III collagen comprises amino acid residue 168 to amino acid residue 1196 of the amino acid sequence of SEQ ID NO:1 or collagenous, e.g., triple helical, fragments thereof.

The invention further provides an implantable composition comprising collagen free of intramolecular and intermolecular crosslinks, wherein the implantable composition is selected from the group consisting of a dermal filler, a bone-void filler, and a dental implant. In specific embodiments, the implantable composition is suitable for use in tissue augmentation. Embodiments in which the tissue augmentation is soft tissue augmentation and in which the tissue augmentation is hard tissue augmentation are specifically contemplated.

In one aspect, the present invention provides a method for augmenting soft tissue in a subject, the method comprising administering to the subject at an augmentation site a composition comprising collagen, wherein the collagen consists of type III collagen, thereby augmenting the soft tissue. The method is not a method for treatment of the human or animal body by surgery or therapy. In one aspect, the subject is a mammal. In a preferred aspect, the subject is a human. In another aspect, the type III collagen is of the same species as the subject. In a particular aspect, the type III collagen is human type III collagen and the subject is a human. In another aspect, the type III collagen is synthetic type III collagen. In a preferred aspect, the type III collagen is synthetic human type III collagen. In certain aspects, the type III collagen comprises the sequence of SEQ ID NO:1 or collageneous, e.g., triple helical, fragements thereof. In other aspects, the type III collagen comprises amino acid residue 149 to amino acid residue 1221 of the amino acid sequence of SEQ ID NO:1 or collagenous, e.g., triple helical, fragments thereof. In yet other aspects, the type III collagen comprises amino acid residue 168 to amino acid residue 1196 of the amino acid sequence of SEQ ID NO:1 or collagenous, e.g., triple helical, fragments thereof.

In various aspects, the administering to the subject at an augmentation site is by implantation or injection, e.g., using a syringe and needle, etc. In certain aspects, the administering to the subject at an augmentation site is subcutaneous administration, intradermal administration, or subdermal administration.

In one aspect, the present invention provides a method for augmenting soft tissue in a subject, the method comprising administering to the subject at an augmentation site a composition comprising collagen, wherein the collagen is free of intermolecular and intramolecular crosslinks, thereby augmenting the soft tissue. The method is not a method for treatment of the human or animal body by surgery or therapy. In one aspect, the subject is a mammal. In a preferred aspect, the subject is a human. In another aspect, the collagen is fibrillar collagen. The collagen is selected from type III collagen. In a preferred aspect, the collagen is human type III collagen. It is contemplated that, in various aspects, the collagen is synthetic collagen. In a particular aspect, the collagen comprises the sequence of SEQ ID NO:1 or collagenous, e.g., triple helical, fragments thereof. In other aspects, the type III collagen comprises amino acid residue 149 to amino acid residue 1221 of the amino acid sequence of SEQ ID NO:1 or collagenous, e.g., triple helical, fragments thereof. In yet other aspects, the type III collagen comprises amino acid residue 168 to amino acid residue 1196 of the amino acid sequence of SEQ ID NO:1 or collagenous, e.g., triple helical, fragments thereof.

. In one embodiment, the present invention provides a method for augmenting soft tissue in a subject, the method comprising administering to the subject at an augmentation site a composition comprising collagen, wherein the collagen is free of endogenous crosslinks, thereby augmenting the soft tissue. The method is not a method for treatment of the human or animal body by surgery or therapy. In one embodiment, the subject is a mammal. In a preferred embodiment, the subject is a human. Collagens free of endogenous crosslinks may include but are not limited to collagens that, while free of endogenous crosslinks, have been exposed subsequent to endogenous production to crosslinking agents (chemical crosslinking agents, e.g., gluteraldehyde, etc.) or to crosslinking conditions (e.g., heat, radiation, etc.), such that the collagen does contain non-endogenously imposed crosslinks. In one embodiment, the collagen is fibrillar collagen, The collagen is selected from type III collagen. In a preferred embodiment, the collagen is human type III collagen. It is contemplated that, in various embodiments, the collagen is synthetic collagen. In a particular embodiment, the collagen comprises the sequence of SEQ ID NO:1 or collagenous, e.g., triple helical, fragments thereof. In other embodiments, the type III collagen comprises amino acid residue 149 to amino acid residue 1221 of the amino acid sequence of SEQ ID NO:1 or collagenous, e.g., triple helical, fragments thereof. In yet other embodiments, the type III collagen comprises amino acid residue 168 to amino acid residue 1196 of the amino acid sequence of SEQ ID NO:1 or collagenous, e.g., triple helical, fragments thereof.

The present invention provides a method for preparing an implantable collagen composition, the method comprising providing a collagen, wherein the collagen consists of type III collagen, and preparing an aqueous solution of the type III collagen, thereby preparing an implantable collagen composition. In one embodiment, the type III collagen is human type III collagen. In another embodiment, the type III collagen is synthetic type III collagen. In a preferred embodiment, the type III collagen is synthetic human type III collagen. In yet another embodiment, the type III collagen is free of any intermolecular and intramolecular crosslinks. In a further embodiment, the type III collagen is free of any endogenous crosslinks. In certain embodiments, the type III collagen comprises the sequence of SEQ ID NO:1 or collagenous, e.g., triple helical, fragments thereof. In other embodiments, the type III collagen comprises amino acid residue 149 to amino acid residue 1221 of the amino acid sequence of SEQ ID NO:1 or collagenous, e.g., triple helical, fragments thereof. In yet other embodiments, the type III collagen comprises amino acid residue 168 to amino acid residue 1196 of the amino acid sequence of SEQ ID NO:1 or collagenous, e.g., triple helical, fragments thereof.

In one embodiment, the present invention provides a method for preparing an implantable collagen composition, the method comprising providing a collagen free of intermolecular and intramolecular crosslinks, and preparing an aqueous solution of the collagen free of intermolecular and intramolecular crosslinks, thereby preparing an implantable collagen composition. In one embodiment, the collagen is fibrillar collagen, and, in particular embodiments, is selected from the group consisting of type III collagen. In certain embodiments, the collagen is human collagen. In other embodiments, the collagen is synthetic collagen. In preferred embodiments, the collagen is synthetic human collagen. In a preferred embodiment, the collagen is synthetic human type III collagen. In a further embodiment, the collagen is substantially free of any endogenous crosslinks. In a particular embodiment, the collagen comprises the sequence of SEQ ID NO:1 or collagenous, e.g., triple helical, fragments thereof. In other embodiments, the type III collagen comprises amino acid residue 149 to amino acid residue 1221 of the amino acid sequence of SEQ ID NO:1 or collagenous, e.g., triple helical, fragments thereof. In yet other embodiments, the type III collagen comprises amino acid residue 168 to amino acid residue 1196 of the amino acid sequence of SEQ ID NO:1 or collagenous, e.g., triple helical, fragments thereof.

In one embodiment, the present invention provides a method for preparing an implantable collagen composition, the method comprising providing a collagen free of endogenous crosslinks, crosslinking the collagen, and preparing an aqueous solution of the crosslinked collagen, thereby preparing an implantable collagen composition. In one embodimment, crosslinking the collagen occurs by exposing the collagen to heat or radiation under conditions suitable to crosslink the collagen. The collagen is fibrillar collagen, and is selected from the group consisting of type III collagen. In another embodiment, the collagen is human collagen. In another embodiment, the collagen is synthetic collagen. In a preferred embodiment, the collagen is synthetic human collagen. In a particular embodiment, the collagen comprises the sequence of SEQ ID NO:1 or collagenous, e.g., triple helical, fragments thereof. In other embodiments, the type III collagen comprises amino acid residue 149 to amino acid residue 1221 of the amino acid sequence of SEQ ID NO:1 or collagenous, e.g., triple helical, fragments thereof. In yet other embodiments, the type III collagen comprises amino acid residue 168 to amino acid residue 1196 of the amino acid sequence of SEQ ID NO:1 or collagenous, e.g., triple helical, fragments thereof. In another embodiment, crosslinking the collagen occurs by exposing the collagen to a chemical crosslinking agent under conditions suitable to crosslink the collagen. In certain embodiments, the chemical crosslinking agent is selected from the group consisting of aldehydes, carbodiimides, epoxides, and imidazoles.

In one aspect, the present invention provides a kit useful for augmenting soft tissue comprising an implantable collagen composition, the kit comprising type III collagen, a syringe, and a needle. In one aspect, the type III collagen is human type III collagen. In another aspect, the type III collagen is synthetic type III collagen. In a preferred aspect, the type III collagen is synthetic human type III collagen. In yet another aspect, the type III collagen is free of any intermolecular and intramolecular crosslinks. In a further aspect, the type III collagen is free of any endogenous crosslinks. In certain aspects, the type III collagen comprises the sequence of SEQ ID NO:1 or collagenous, e.g., triple helical, fragments thereof. In other aspects, the type III collagen comprises amino acid residue 149 to amino acid residue 1221 of the amino acid sequence of SEQ ID NO:1 or collagenous, e.g., triple helical, fragments thereof. In yet other aspects, the type III collagen comprises amino acid residue 168 to amino acid residue 1196 of the amino acid sequence of SEQ ID NO:1 or collagenous, e.g., triple helical, fragments thereof.

### BRIEF DESCRIPTION OF THE DRAWINGS

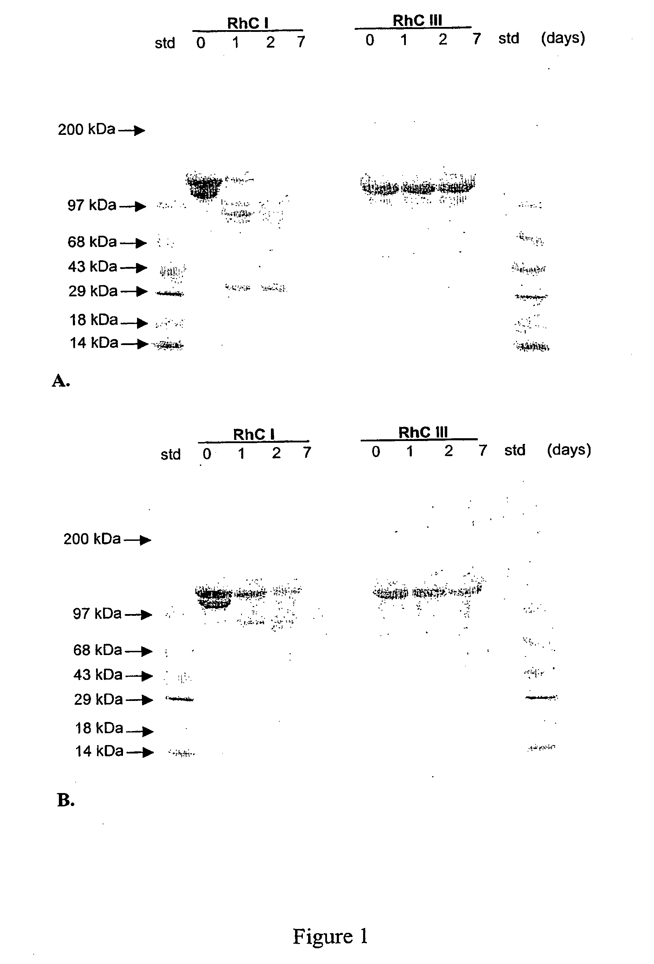

Figures 1A and 1B show SDS-PAGE analysis of synthetic human type I collagen and synthetic human type III collagen following treatment with matrix metalloproteinase (MMP)-1 (Figure 1A) and MMP-δ (Figure 1B).

### DESCRIPTION OF THE INVENTION

The present invention provides implantable collagen compositions useful for tissue augmentation applications as described in claim 1. The implantable collagen compositions of the present invention have increased persistence suitable for use in various medical and cosmetic applications. Implantable collagen compositions with minimal or no variability and with improved manipulability, extrudability, and intrudability are also provided. The invention also provides the method described in claim 10.

The present invention relates in part to the discovery that type III collagen displays greater persistence than does type I collagen. In particular, the present inventors discovered that implantable type III collagen compositions have persistence greater than that of implantable type I collagen compositions, e.g., type III collagen will persist longer and degrade at a slower rate than type I collagen. Therefore, in one embodiment, the present invention provides an implantable composition comprising collagen, wherein the collagen consists of type III collagen.

The determination of whether the amount of type III collagen in a particular composition is sufficient to give that composition increased persistence can be measured, e.g., by evaluating the longevity of a composition containing a specific percentage of type III collagen, such as by visual or palpable assessment, for example, using Global Aesthetic Improvement Scale (GAIS) ratings, or by assessing *in vitro* resistance to metalloprotease degradation (see, e.g., Example 2), etc. GAIS is based on a physician's assessment of the overall improvement, e.g., cosmetic improvement, in a treated area, e.g., nasolabial fold, by comparing the patient's appearance after treatment to that before treatment. GAIS ratings include: very much improved (optimal cosmetic result for the implant in the patient); much improved (marked improvement in appearance from the initial condition, but not completely optimal for this patient); improved (obvious improvement in appearance from initial condition); no change (the appearance is essentially the same as the original condition); and worse (the appearance is worse than the original condition).

No current products provide a collagen component that consists of type III collagen or that consists of a predetermined and reproducible amount of type III collagen sufficient to provide increased persistence. Collagen used as a tissue bulking or augmenting material in various procedures is endogenous collagen, either extracted from animal, typically bovine tissue, e.g., by dispersion (mechanically shearing the tissue), dissolution (acid cleavage), enzymatic digestion, etc., or endogenously produced, e.g., by cultured fibroblasts. Endogenous collagen is a variable mixture of collagen types, typically containing 95% type I collagen, e.g., the predominant collagen type in bones and tissues, approximately 5% type III collagen, as well as trace amounts of other collagen types.

Endogenous human collagen can be similarly obtained from skin derived from the patient or from cadavers. (See, e.g., U.S. Patent Number 4,969,912 and U.S. Patent Number 5,116,389.) Like tissue-extracted bovine collagen, extracted human collagen is a mixture of collagen types; for example, human skin collagen contains about 95% type I collagen, 5% type III collagen, and trace amounts of other collagen types. The amounts of collagen thus obtained are limited, and cost and consumer distaste are prohibitive. Human collagen has also been obtained from human fibroblast cells grown in a controlled laboratory environment. (See, e.g., COSMODERM and COSMOPLAST formulations, Inamed Corp., Santa Barbara CA.) These cultured fibroblasts behave as if they were in the human body, and produce natural collagen, e.g., the collagen normally found in human skin, e.g., a mixture of approximately 95% type I collagen, 5% type III collagen, and trace amounts of other collagen types. The collagen thus obtained is endogenous material, and production varies based on growth conditions and occurs in relatively limited amounts.

The present invention further relates to the unexpected finding that collagen compositions containing collagen free of intermolecular and intramolecular crosslinks are useful as implantable materials.

All collagen currently used in tissue augmentation products is endogenous collagen, e.g., extracted from animal, e.g., bovine or human, tissues or endogenously produced, for example, by cultured fibroblasts. Endogenous collagens are subject to extensive post-translational modifications. (Kivirikko (1998) Matrix Biol 16:355-356.) In particular, the assembly and processing of collagens, such as the fibrillar collagens (e.g., type I, type II, and type III collagen), is accompanied by the formation of intramolecular and intermolecular covalent crosslinks, i.e., crosslinks that form intramolecularly between the individual collagen α-chains forming a triple helical collagen molecule, or intermolecularly between triple helical collagen molecules, enhancing fibrillar stability. Natural crosslinks form between hydroxylysine and lysine residues or between hydroxylysine, lysine, and histidine residues of the collagen molecules.

Crosslinks confer tensile and mechanical strength and contribute to tissue integrity and are associated with resistance of the collagen to degradation by host collagenases, proteases, etc. Therefore, it has been believed in the art that a degree of natural or endogenous crosslinking was required in order for collagen to be useful as an implant material having an appropriate amount of mechanical and structural function and persistence for use in various medical and cosmetic applications. Crosslinking contributes to and prolongs the structural integrity of crosslinked collagen, but also affects other physical parameters, including flowability and solubility, and limits the workable concentration of collagen in a final tissue augmentation product. Therefore, the ease of manipulation and handling of the collagen, and its suitability for injection, can be compromised by the degree and nature of crosslinking present.

The present invention shows surprisingly that collagen free of crosslinks, e.g., substantially free of intermolecular and intramolecular crosslinks, demonstrated persistence and resistance to degradation and thus suitability for use in various tissue augmentation applications. (See Example 2 and Example 3.) Therefore, in one embodiment, the present invention provides an implantable composition containing collagen, wherein the collagen is free of intramolecular and intermolecular crosslinks. This offers unique advantages over currently available products, as collagen free of intermolecular or intramolecular crosslinks is not affected by the loss of or reduction in manipulability, extrudability, and intrudability characteristic of crosslinked material. In one embodiment, the collagen is a fibrillar collagen. The collagen is selected from the group consisting of type III collagen

While the present invention provides implantable compositions comprising collagen free of intramolecular and intermolecular crosslinks, implantable compositions comprising collagens free of endogenous crosslinks, but containing non-endogenously imposed crosslinks, are specifically contemplated herein.

As crosslinks have been traditionally associated with increased stability and persistence of implanted collagen material, various current collagen-containing products involve the use of chemically or other exogenously imposed crosslinks to further enhance the persistence of the collagen material. (See McPherson et al. (1986) J Biomed Mater Res 20:93-107.) For example, clinical studies using a commercially available injectable bovine collagen (i.e., ZYDERM) demonstrated that, while efficacious at correcting facial wrinkles, the clinical correction was temporary. (See Burke et al. (1985) Ann Plast Surg 14:515-522; Rapaport et al. (1984) J Am Acad Dermatol 11(2 Pt 1):25-252.) This prompted the development of the more stable injectable material ZYPLAST, a gluteraldehyde crosslinked injectable bovine collagen, which is a more persistent material than ZYDERM and thus provides a more durable aesthetic effect. Therefore, as taught in the art, implantable biomaterials containing exogenously imposed crosslinks are associated with a more stable and durable material and with increased persistence of the material.

These additional or non-endogenous crosslinks are introduced to the collagen material, e.g., by exposing the collagen to chemical crosslinking agents, radiation, etc. As noted, *supra*, an increased degree of crosslinking may enhance persistence but can compromise other aspects of performance of the collagen material, including ease of handling, such that in some cases the collagen requires additional processing and treatment to render it suitable for injection. (See, e.g., U.S. Patent No. 4,582,640.)

It is an advantage of the present material that, in one aspect, the present invention provides a starting collagen material free of any intramolecular or intermolecular crosslinks. This collagen can be exposed to crosslinking agents or conditions suitable to induce crosslinking (e.g., gluteraldehyde, radiation, etc.), resulting in collagen possessing a degree of crosslinking that is sufficient to enhance persistence, but that does not compromise the ease of handling of the material. In particular, a collagen that is free of any endogenous intermolecular or intramolecular crosslinks, and that is subsequently crosslinked, e.g., by use of chemical agent or exposure to crosslinking conditions such as radiation, can be crosslinked to a lesser extent than an endogenously crosslinked collagen subsequently crosslinked under the same conditions.

In one aspect, the invention provides an optimal starting material for manufacture of an implantable collagen composition, wherein the starting material is or comprises a collagen free of endogenous crosslinks. While the invention specifically contemplates, in various aspects, that the manufacture of an implantable composition suitable for use in various cosmetic and medical procedures, including tissue augmentation procedures, will involve crosslinking the collagen, it is a distinct advantage of the particular compositions of the present invention that the degree and nature of the crosslinking imposed are completely within control of the manufacture. In contrast, the endogenously produced materials currently available contain endogenous crosslinks as a result of their natural production. The present invention thus, in certain embodiments, offers uniquely consistent and reproducible starting material, allowing for a predictable and controlled performance at every step in manufacturing and offering advantages over the endogenously produced materials used today.

Thus, in one aspect, the invention contemplates a method of manufacturing an implantable collagen composition, the method comprising: obtaining collagen free of any intramolecular or intermolecular crosslinks, and crosslinking the collagen, thereby manufacturing an implantable collagen composition. Collagen can be crosslinked using methods known to one of skill in the art, such as by heat, radiation, or using chemical crosslinking agents available in the art, such as, for example, aldehydes, carbodiimides, epoxides, imidazoles, etc.

This is advantageous in that is allows more control and predictability with respect to the nature and degree of crosslinking of the collagen. In particular, the degree of crosslinking can be controlled, e.g., by modification of crosslinking agent concentration or radiation strength, or time of exposure to the modifying conditions. The nature, including the type, of crosslinking can be controlled, e.g., by altering conditions to favor the formation of intramolecular over intermolecular crosslinks, such as by use of particular crosslinking agents, by varying the concentration of collagen in the solution to be crosslinked, etc.

In one embodiment, the present invention provides an injectable composition comprising collagen with no endogenous crosslinks. This can include both a material that contains no intermolecular and no intramolecular crosslinks, and a material that contains no endogenous crosslinks, but does contain crosslinks introduced in a non-endogenous setting. The crosslinks introduced in a non-endogenous setting include, e.g., crosslinks resulting from exposure of the collagen to crosslinking agents, such as chemical crosslinking agents, from exposure of the collagen to radiation, etc.

In preferred embodiments, collagen used in the present compositions and methods is produced by synthetic means, most preferably by recombinant means. This can be advantageous for a number of reasons, as the use of synthetic collagen in an implantable composition of the present invention results in a characterized and fully reproducible collagen that has sufficient strength, stability, and, persistence, essentially no immunoreactivity, tissue response, or inflammatory reaction upon implantation, and that is free of pathogenic, e.g., infectious, agents, and of other contaminants, e.g., animal-derived contaminants, etc.

The use of bovine or other animal collagen (porcine, equine, etc.) as the predominant component of various tissue augmentation and implant materials carries with it the potential for adverse reaction upon implantation/injection. In particular, immune reponses including allergic reactions and non-allergic reactions, e.g., infections, etc., have been observed upon exposure to non-human collagen-containing products. (See, e.g., Baumann and Weisberg, *supra*, page 157.) For example, positive allergic reactions to bovine collagen are seen in 3 to 10 percent of patients undergoing skin testing. (See Barr and Stegman (1984) J Am Acad Dermatol 10: 652; Charrierre et al. (1989) J Am Acad Dermatol 21:1203.) There is also a smaller but significant subgroup of allergic patients who develop an allergy to bovine collagen even after one or two negative skin tests. These reactions can be devastating and difficult, if not impossible, to treat.

If an inflammatory response is stimulated, by exposure to an allergenic, immunogenic, or even toxic component present in the collagen source material, infiltrating inflammatory cells and fibroblasts can restructure both native and implanted matrix components, and the injected/implanted collagen can be degraded and resorbed. Therefore, in one embodiment, the implantable compositions of the present invention comprise synthetic collagen. In a further embodiment, the synthetic collagen is recombinantly manufactured, for example, produced as described, e.g., in Example 1.

In addition to minimizing the risk for adverse reactions, the use of synthetic collagen in the implantable compositions of the present invention can be advantageous in contributing to the uniformity and reproducibility of the collagen material. As discussed, supra, endogenous collagens, including those extracted from natural sources, contain a heterogenous mixture of various types of collagen. (See, e.g., Byers et al. (1974) Biochemistry 13:5243-5248; and Miller and Rhodes (1982) Methods Enzymol 82 (Pt A):33-64.) In contrast, the present invention clearly contemplates implantable compositions containing collagen of one type free of any other type of collagen, or compositions comprising specific and predetermined percentages of more than one collagen type. In a preferred embodiment, the present invention provides an implantable composition comprising collagen, wherein the collagen consists of type III collagen. In other embodiments, the invention provides implantable compositions containing collagen, wherein the collagen comprises a specific and predetermined percentage of type III collagen. Use of synthetic, including recombinant, techniques, is advantageous in producing individual collagen types in isolated and predetermined quantities and in reproducible fashion.

Furthermore, the use of synthetic techniques enables the production of collagens that can be specifically optimized for particular uses. For example, expression constructs encoding synthetic collagens suitable for use in the present invention can contain native human sequence but have specific modifications that result in expression of synthetic collagens having desired properties, e.g., deletions, alterations, substitutions, etc., that eliminate collagenase-sensitive regions, and deletions, alterations, substitutions, etc., of various regions eliciting immunogenic, e.g., antigenic and allergenic, responses, etc.

Synthetic collagens can be produced using any of the wide range of synthetic techniques well-known in the art, including, e.g., recombinant production, peptide synthesis, etc. A preferred recombinant method is described in, e.g., Vuorela et al. (1997) EMBO J 16:6702-6712; Nokelainen et al. (2001) Yeast 18:797-806; and Myllyharju et al. (2000) Biochem Soc Trans 28:3532-3537, incorporated by reference herein in their entirety. For recombinant production, the host cells are not restricted, and can include any cell capable of recombinant protein expression. Upon expression, the synthetic collagens may be secreted into the media or local environment, and/or the host cell may retain the collagen. Retained collagen may be compartmentalized, for example in the endoplasmic reticulum, golgi, or associated vesicles in animal cells, vacuoles in plant cells, inclusion bodies in bacteria, etc. The cells expressing the synthetic collagen may be grown in culture, or may be inserted by techniques known to those skilled in the art into transgenic plants or animals and then isolated from tissues targeted for expression.

Collagen sequences from which synthetic collagens can be derived include animal collagens and, in preferred embodiments, human collagens. Nucleic acid sequences encoding collagens are available in the art. (See, e.g., Fuller and Boedtker (1981) Biochemistry 20:996-1006; Sandell et al. (1984) J Biol Chem 259:7826-34; Kohno et al. (1984) J Biol Chem 259:13668-13673; French et al. (1985) Gene 39:311-312; Metsaranta et al. (1991) J Biol Chem 266:16862-16869; Metsaranta et al. (1991) Biochim Biophys Acta 1089:241-243; Wood et al. (1987) Gene 61:225-230; Glumoff et al. (1994) Biochim Biophys Acta 1217:41-48; Shirai et al. (1998) Matrix Biology 17:85-88; Tromp et al. (1988) Biochem J 253:919-912; Kuivaniemi et al. (1988) Biochem J 252:633-640; Ala-Kokko et al. (1989) Biochem J 260:509-516; International Publication No. WO 01/34647.) Further, the collagens used in the present formulations may comprise collagen fragments of defined length produced by direct recombinant expression of polynucleotides encoding the collagen fragment, or collagen fragments obtained by processing full-length collagen protein, e.g., by enzymatic digestion, thermal denaturation, chemical degradation, etc. Collagen fragments can include any polypeptide comprising at least a portion of the triple helical domain of a collagen or collagen-like molecule. In a preferred embodiment, the present formulations comprise synthetic collagen or synthetic collagen fragments derived from type III collagen; in a most preferred embodiment, from human type III collagen. Each synthetic collagen produced by the methods described above can be obtained as a single type of collagen, free of any other type of collagen.

In one embodiment, the present invention provides an implantable composition containing collagen, wherein the collagen is type III collagen. In another embodiment, the type III collagen is human type III collagen. In one embodiment, the present invention provides a collagen comprising the amino acid sequence of SEQ ID NO:1 or a collagenous fragment thereof. In one embodiment, the collagen comprises a polypeptide having an amino acid sequence containing the N-telopeptide domain, the α-helical domain, and the C-telopeptide domain of type III collagen. The N-telopeptide of type III collagen is from amino acid residue 149 to amino acid residue 167 of SEQ ID NO:1. The α-helical domain of type III collagen is from amino acid residue 168 to amino acid residue 1196 of SEQ ID NO:1. The C-telopeptide of type III collagen is from amino acid residue 1197 to amino acid residue 1221 of SEQ ID NO:1. In one embodiment, the collagen useful in the present methods and compositions comprises a polypeptide having an amino acid sequence of amino acid residue 149 to amino acid residue 1221 of SEQ ID NO:1. In another embodiment, the collagen has an amino acid sequence of from about amino acid residue 149 to amino acid residue 1221 of SEQ ID NO:1. In addition, an implantable composition containing collagen, wherein the collagen is a synthetic human type III collagen which contains an isoleucine to proline substitution at amino acid residue 1205 of the human type III collagen sequence of SEQ ID NO:1, is specifically contemplated herein.

In another embodiment; the collagen comprises a polypeptide having an amino acid sequence containing the α-helical domain of type III collagen. In one embodiment, the collagen useful in the compositions and methods of the present invention comprises a polypeptide having an amino acid sequence of from amino acid residue 168 to amino acid residue 1196 of SEQ IOD NO:1. It is further contemplated that in certain embodiments of the present invention, the collagen for use in the present methods and compositions comprises a polypeptide having an amino acid sequence of about amino acid residue 168 to amino acid residue 1196 of SEQ ID NO:1.

The implantable compositions provided herein can be used in any method known or contemplated by those skilled in the art. In particular, the present compositions can be used in any of the numerous medical and cosmetic applications, including tissue augmentation procedures, in which collagen is currently used and in which compositions containing collagen and having greater persistence, improved handling, and/or less variability may be desired. The present compositions are suitable for use in tissue augmentation procedures. Use of the present compositions in cosmetic as well as in medical procedures is specifically contemplated. The scope of the invention is defined in the appended claims.

In one aspect, the present invention provides implantable compositions containing collagen and suitable for use in soft tissue augmentation procedures. The present compositions can be implanted or injected into various regions of the skin or dermis, depending on the particular application or cosmetic procedure, including dermal, intradermal, and subcutaneous injection or implantation. The materials of the present invention can also be injected or implanted superficially, such as, for example, within the papillary layer of the dermis, or can be injected or implanted within the reticular layer of the dermis.

In addition to soft tissue augmentation, use of the implantable compositions containing collagen for hard tissue augmentation is contemplated in the present invention. The present collagen compositions are useful in various hard tissue augmentation applications, including, for example, as a bone-void filler, dental implant, etc.

Cosmetic uses of the compositions of the present invention include treatment of fine lines, such as fine superficial facial lines, wrinkles, and scars, as well as treatment of pronounced lines, wrinkles, and scars. In some aspects, the compositions of the present invention are used for other cosmetic uses, including treatment for or reducing transverse forehead lines, glabellar frown lines, nasolabial fold, vermilion border, periorbital lines, vertical lip lines, oral commissure, etc., as well as defining the lip border. The compositions of the present invention are also useful for correcting contour deformities and distensible acne scars, or for treating other tissue defects, such as, for example, atrophy from disease or trauma or surgically-induced irregularities.

In certain embodiments, the injectable compositions of the present invention are used for surgical procedures involving tissue augmentation, tissue repair, or drug delivery. In some aspects, the compositions are used for tissue augmentation in conditions such as urinary incontinence, vasicoureteral reflux, and gastroesophageal reflux. For example, compositions of the present invention may be used to add tissue bulk to sphincters, such as a gastric or urinary sphincter, to provide proper closure and control. In instances of urinary incontinence, such as stress incontinence in women or incontinence following a prostatectomy in men, the compositions of the invention may be provided to further compress the urethra to assist the sphincter muscle in closing, thus avoiding leakage of urine from the bladder.

Similarly, gastroesophageal reflux disease (GERD, also known as peptic esophagitis and reflux esophagitis) is a disorder that affects the lower esophageal sphincter, the muscle connecting the esophagus with the stomach. GERD occurs when the lower esophageal sphincter is incompetent, weak, or relaxes inappropriately, allowing stomach contents to flow up into the esophagus (i.e., reflux). Malfunction of the lower esophageal sphincter muscles, such as that resulting from muscle tonal loss, can lead to incomplete closure of the lower esophageal sphincter, causing back up of acid and other contents from the stomach into the esophagus. Poor response to dietary modification or medical treatment may require surgery to correct the dysfunction. In one embodiment, collagen compositions of the present invention are used in such procedures and, for example, are injected into the area of the esophageal sphincter to provide bulk to the lower esophageal sphincter.

In other embodiments, the implantable collagen compositions of the invention are used to fill or block voids and lumens within the body. Such voids may include, but are not limited to, various lesions, fissures, diverticulae, cysts, fistulae, aneurysms, or other undesirable voids that may exist within the body; and lumens may include, but are not limited to, arteries, veins, intestines, Fallopian tubes, and trachea. For example, an effective amount of the present composition may be administered into the lumen or void to provide partial or complete closure, or to facilitate repair of damaged tissue.

In other aspects, tissue repair is achieved by providing the collagen composition of the present invention to an area of tissue that has been diseased, wounded, or removed. In some embodiments, compositions of the invention are used to fill in and/or smooth out soft tissue defects such as pockmarks or scars. In such cases, a formulation of the present invention is injected beneath the imperfection. The improved persistence of the present compositions would be beneficial, e.g., by reducing the number and frequency of treatments required to obtain a satisfactorily result. In certain embodiments, the collagen compositions are used for intracordal injections of the larynx, thus changing the shape of this soft tissue mass and facilitating vocal function. Such use is specifically contemplated for the treatment of unilateral vocal cord paralysis. Further, the present invention contemplates use of the collagen compositions in mammary implants, or to correct congenital anomalies, acquired defects, or cosmetic defects.

The present collagen compositions can also be used in various surgical or other procedures for remodeling or restructuring of various external or internal features, e.g., plastic surgery for corrective or cosmetic means, etc.

In any of the embodiments described above, the present compositions may be used for drug delivery, for example, to deliver drugs to an injection site. The drugs can be delivered in a sustained manner from an *in vivo* depot formed by the collagen upon injection of an implantable composition of the present invention. Drugs delivered in this manner may thus enhance tissue repair, and could provide additional therapeutic benefit.

In additional embodiments, the invention further contemplates incorporation of cells into the implantable collagen compositions to provide a means for delivering cells to repopulate a damaged or diseased tissue or to provide products synthesized by the cells to the tissues surrounding the injection site.

In any of the embodiments described above, the implantable compositions of the present invention may be delivered or administered by any suitable method known or contemplated by those of skill in the art. The invention specifically contemplates delivery by injection, e.g., using a syringe. In some embodiments, the collagen compositions may additionally contain a biocompatible fluid that functions as a lubricant to improve the injectability of the formulation. The collagen compositions of the invention can be introduced into the tissue site by injection, including, e.g., intradermal, subdermal, or subcutaneous injection.

It is understood that the compositions of the present invention can include additional components suitable to the particular formulation. For example, in certain embodiments, the implantable compositions of the present invention are intended for injection and are formulated in aqueous solutions. The compositions can be formulated to include pharmaceutically acceptable carriers and excipients. Such carriers and excipients are well-known in the art and can include, e.g., phosphate buffered saline (PBS) solutions, various solvents, and salts, etc., for example, physiologically compatible buffers including physiological saline buffers such as Hanks's solution and Ringer's solution.

The amount of collagen appropriately included in a particular formulation is determined as standard in the art for such formulations, and is dictated by the intended use. In certain embodiments, the present invention provides implantable compositions comprising collagen wherein the collagen is in aqueous solution at a concentration between about 20 to about 120 mg/ml. In some embodiments, the collagen is in aqueous solution at a concentration between about 30 to about 90 mg/ml; or a concentration of between about 20 to 65 mg/ml; or a concentration of between about 25 to 40 mg/ml. In preferred embodiments, the collagen compositions of the present invention have a collagen concentration of about 35 mg/ml or a collagen concentation of about 65 mg/ml.

### EXAMPLES

The invention will be further understood by reference to the following examples, which are intended to be purely exemplary of the invention. These examples are provided solely to illustrate the claimed invention. The present invention is not limited in scope by the exemplified embodiments, which are intended as illustrations of single aspects of the invention only. Any methods that are functionally equivalent are within the scope of the invention. Various modifications of the invention in addition to those described herein will become apparent to those skilled in the art from the foregoing description and accompanying figures. Such modifications are intended to fall within the scope of the appended claims.

### Example 1: Production of Synthetic Collagen

Synthetic collagens were produced using known methods, as described in, e.g., Vuorela et al. (1997) EMBO J 16:6702-6712 and Nokelainen et al. (2001) Yeast 18:797-806, each of which is incorporated by reference herein in its entirety, with modifications as described below.

Production of other collagens suitable for use in the present compositions can be specifically engineered using molecular biology techniques know to one of skill in the art. Such collagens can be modified by, e.g., an alteration in the polypeptide coding sequence, including deletion, substitutions, insertions, etc., to increase resistance to degradation. For example, synthetic collagens with alterations in the amino acid sequence at specific protease cleavage sites can be produced. An exemplary collagen for use in the present compositions is a synthetic human type III collagen which contains an isoleucine to proline substitution at amino acid residue 1205 of the human type III collagen sequence of SEQ ID NO:1.

Synthetic human type III collagen was prepared essentially as described previously (Vuorela et al. (1997) EMBO J 16:6702-6712). A polynucleotide encoding human α1 (III) procollagen was used. Linearized plasmid containing a polynucleotide encoding human α1(III) procollagen was resuspended in deionized(di)H₂O at ∼1 µg/mL and electroporated into a *Pichia pastoris* strain that expresses the α and β subunits of human prolyl hydroxlase (Vuorela et al. (1997) EMBO J 16:6702-6712). A selected yeast strain was grown in a 100L fermentation tank in basal salt glycerol medium, pH 5. The strain was grown in the fed-batch mode on glycerol until the wet cell weight reached 215 g/L (6 hours). The glycerol feed was changed to methanol feed for 110 hours, to a final wet cell weight of 380 g/L. Cells were washed with water, lysed in citric acid buffer, and incubated for 48 hours to release synthetic human type III collagen from the cells.

Pepsin was added to the collagen (i.e., procollagen) solution to remove N- and C-propeptide regions from the synthetic collagen. The non-specific nature of pepsin cleavage of collagen resulted in the N- and C-telopeptide regions having variable lengths, as known in the art The synthetic human type III collagen was then recovered by acid salt precipitation. The precipitated type III collagen was dissolved in 0.1M HCl and clarified by centrifugation and filtration. The collagen solution was then adjusted to pH 5.8 with Tris base in order to precipitate more impurities, then clarified by centrifugation and filtration through a 0.2 µm membrane. The collagen solution was adjusted to pH 9.0 with Tris base and to a final concentration of 1M urea, and then passed over a DEAE-Sepharose FF column (6L). Synthetic human type III collagen was precipitated from the DEAE flowthrough by addition of HCl and NaCl. The precipitate was concentrated by centrifugation and dissolved in 0.1M HCl. The collagen solution was then clarified by 0.2µm membrane filtration and diafiltered into 10 mM HCl. The collagen solution was then adjusted to pH 7.2 by adding 1/10 volume of 0.2M Na-PO4, pH 11.2, and incubated overnight to precipitate the collagen. Precipitated synthetic human type III collagen was collected by centrifugation, dissolved in 0.1M HCl, and filtered through a 0.2µm membrane filter. The filtrate was diafiltered into 10 mM HCl, sterile filtered through a 0.2µm membrane filter, and stored at 4-8°C.

Collagen fibrils were formed from the synthetic human type III collagen and synthetic human type I collagen as follows. Fibrillogenesis buffer (0.2 M Na₂HPO₄, pH 11.2) was added to a 0.3% (3 mg/ml) solution of synthetic human type III collagen and of synthetic human type I collagen at a 1:10 (v/v) ratio. The solution was incubated at room temperature from 4 hours to overnight. Synthetic human type III collagen fibrils and synthetic human type I collagen fibrils were then collected by centrifugation at 15,000 x g for 30 minutes at 10°C.

The pellet was resuspended in PBS to the original volume, and then centrifuged as before. The resultant pellet was transferred to a 10 cc syringe (Becton, Dickinson and Co., Franklin Lakes NJ) and connected via a Luer Lok-to-Luer Lok connector (Baxa, Englewood CO) to a second 10 cc syringe containing 1/5^{th} volume 0.9% NaCl (Irrigation USP; Braun-McGaw, Detroit MI). The synthetic collagen was transferred into the second syringe through the connector, air bubbles were removed from the resulting collagen suspension, and then the synthetic collagen was transferred approximately 20 times between the syringes. The connector was then replaced with an 18G micro-emulsifying needle (Popper Precision Instruments, Lincoln RI) and the synthetic collagen was passed through the needle approximately 20 times to further homogenize the material. The mixture was then adjusted to 35 mg/ml collagen and 3 mg/ml lidocaine (lidocaine hydrochloride, USP; Wyckoff Chemical Co, Inc., South Haven MI) in 0.9% PBS and the collagen mixture was passed through an 18G needle as before. Final homogenized synthetic collagen was stored at 4°C.

### Example 2: Persistence of synthetic human collagen in vitro

The effects of collagenases on synthetic human type III collagen and synthetic human type I collagen, each substantially free of intermolecular and intramolecular crosslinks, were examined. Matrix metalloproteinases (MMPs), also referred to as collagenases, are enzymes capable of cleaving triple-helical collagen. Both MMP-1 (collagenase 1, fibroblast collagenase) and MMP-8 (collagenase 2, neutrophil collagenase) cleave triple-helical collagen types I, II, and III.

Synthetic human type III collagen was prepared as described above in Example 1. MMP-1 pro-enzyme and MMP-8 were obtained from EMB Biosciences (San Diego, CA). MMP-1 was converted from the pro-enzyme to the active enzyme according to protocols supplied by the manufacturer. Separate reactions were set up for each MMP used. One milliliter volumes of 2 mg/mL collagen, 50 mM Tris-HCl (pH 7.0), 300 mM NaCl, 5 mM CaCl₂, 0.001 mM ZnCl₂, 0.05% Brij-35, and 0.05% NaN₃ were incubated at 37 °C for one hour. A 200 µL volume was removed from each reaction, and then 0.5 µg MMP-1 or MMP-8 (100-500 mU/mg activity) was added, and the reactions were again incubated at 37°C. Aliquots of 200 µl were removed from each reaction after each of 1, 2, and 7 days incubation. Each 200 µl aliquot was quenched over 20% ethanol/10% trichloroacetic acid and then centrifuged to pellet. The pellets were resuspended in 200 µL 10 mM HCl and fractionated by SDS-PAGE to determine the extent of degradation of the synthetic collagens. The results are shown in Figures 1A and 1B.

As can be seen in Figures 1A and 1B, both synthetic human type I and type III collagen were cleaved by MMP-1 (Figure 1A) and MMP-8 (Figure 1B). However, the rate and extent of MMP degradation of synthetic human type I collagen was substantially higher than that of synthetic human type III collagen for both MMP-1 and MMP-8 enzymes.

For synthetic human type III collagen, the rate of cleavage was slower than that observed for synthetic human type I collagen. Synthetic human type III collagen was degraded by MMP-1 to a greater extent than by MMP-8, which is expected since MMP-1 has a higher specificity for type III collagen. This data showed that collagens substantially free of intermolecular and intramolecular crosslinks demonstrated a certain degree of resistance to degradation by collagenases. These results suggested that collagen substantially free of intermolecular and intramolecular crosslinks can suitably be used in various tissue augmentation procedures. The results further demonstrated that type III collagen is more resistant to degradation by collagenases than is type I collagen, indicating that type III collagen has a higher degree of persistence than does type I collagen.

### Example 3: Persistence of synthetic human collagen in vivo

*In vivo* persistence of implanted synthetic human type I collagen and synthetic human type III collagen was investigated as follows. Wistar rats (Charles River Laboratories, Inc.) were shaved and an 8 cm x 6 cm site for injection was marked the day prior to implantation. Purified collagen preparations in 10 mM HCl at a concentration of 3 mg/mL were mixed with 1/10^{th} volume of 0.2 M NaPO₄ pH 11. The collagen fibrils were collected by centrifugation at 10,000 x g for 15 minutes at 4°C, resuspended in PBS at a collagen concentration of 35 mg/ml, and lidocaine was added to a final concentration of 3 mg/ml.

Implants were made by subcutaneous injection of 0.5 ml of a 35 mg/ml suspension of synthetic human type I collagen, synthetic human type III collagen, or bovine type I collagen (VITROGEN, Cohesion Technologies) in PBS on the dorsal flank. The collagen suspension was injected using a 1 cc syringe with a 28-gauge needle. Each animal received three separate injections of collagen material. For each collagen material tested, a total of 9 implants (3 rats, 3 implants/rat) were present at the start of the study. Groups of three animals I per test material were analyzed at the following time points post implantation: day 2, and week 4, 8, 13, 26, 39, and 52 post implantation. Implants were surgically removed and dissected free from surrounding tissue, and examined macroscopically for appearance and texture. Essentially no inflammatory or tissue response was observed following implantation of either synthetic human collagen.

As is standard in the art, the persistence of the collagen implants was evaluated by determining the number of the original implants that were present at the injection sites at each of the time points. The data are summarized in Table 1 below and are presented as the number of the total implants remaining/number of the original implants at each time point. Surprisingly, all original implants containing type I collagen substantially free of intramolecular and intermolecular crosslinks and all original implants containing type III collagen substantially free of intramolecular and intermolecular crosslinks were present through week 13. Also surprisingly, the implants containing type III collagen showed persistence markedly greater than those of the implants containing type I collagen, and more than half of these implants remained at 52 weeks.

**TABLE 1**

| Time Point | Synthetic human type I collagen | Synthetic human type III collagen |
|---|---|---|
| Day 2 | 9/9 | 9/9 |
| Week 4 | 9/9 | 9/9 |
| Week 8 | 9/9 | 9/9 |
| Week 13 | 9/9 | 9/9 |
| Week 26 | 3/9 | 6/9 |
| Week 39 | 1/9 | 5/9 |
| Week 52 | 1/9 | 5/9 |

These results showed that, first, collagen free of intermolecular and intramolecular crosslinks demonstrated unexpected persistence upon implantation, and is thus suitable for use in various tissue augmentation applications. Additionally, these results demonstrate that type III collagen was more persistent than type I collagen, and that compositions containing exclusively type III collagen can provide unexpected benefits, e.g., enhanced persistence.

## Claims

1. An implantable composition comprising collagen for use in medicine, wherein the collagen consists of type III collagen and the composition is for use in tissue augmentation.

2. The implantable composition of claim 1 for the use of that claim, wherein the type III collagen is human type III collagen or synthetic type III collagen.

3. The implantable composition of claim 1 or claim 2 for the use of that claim, wherein the implantable composition is selected from the group consisting of a dermal filler, a bone-void filler, and a dental implant.

4. The implantable composition of any preceding claim for the use of that claim,
wherein the tissue augmentation is soft tissue augmentation or hard tissue augmentation.

5. The implantable composition of any preceding claim for the use of that claim,
wherein the collagen is a) free of intermolecular and intramolecular crosslinks; or b) free of endogenous crosslinks.

6. The implantable composition of any preceding claim for the use of that claim,
wherein the type III collagen comprises: a) the amino acid sequence of SEQ ID NO:1 or triple helical fragments thereof; b) amino acid residue 149 to amino acid residue 1221 of the amino acid sequence of SEQ ID NO: 1 or triple helical fragments thereof; c) amino acid residue 168 to amino acid residue 1196 of the amino acid sequence of SEQ ID NO: 1 or triple helical fragments thereof; or d) either of a) or b) further comprising an isoleucine to proline substitution at amino acid residue 1205 of SEQ ID NO:1.

7. The implantable composition of claim 4 for the use of that claim, wherein the tissue augmentation is soft tissue augmentation and the composition is for use in a human subject.

8. The composition of any preceding claim for the use of that claim, wherein the composition is for administration by injection and/or for administration by a method selected from the group consisting of subcutaneous administration, intradermal administration, and subdermal administration.

9. The composition of any preceding claim, wherein the composition is for a) use in the treatment of fine lines, wrinkles and scars and pronounced lines, wrinkles and scars; b) use in treating or reducing transverse forehead lines, glabellar frown lines, nasolabial fold, vermilion border, periorbital lines, vertical lip lines, oral commissure, or for defining the lip border; or c) use in correcting contour deformities and distensible acne scars, or for treating tissue atrophy from disease or trauma or surgically-induced tissue irregularities.

10. A method for tissue augmentation comprising the use of an implantable composition comprising collagen, wherein the collagen consists of type III collagen and the method is not a method for treatment of the human or animal body by surgery or therapy.

11. The method of claim 10, wherein the type III collagen is human type III collagen or synthetic type III collagen.

12. The method of claim 10 or claim 11, wherein the implantable composition is a dermal filler.

13. The method of any of claims 10-12, wherein the tissue augmentation is soft tissue augmentation.

14. The method of any of claims 10-13, wherein the collagen is a) free of intermolecular and intramolecular crosslinks; or b) free of endogenous crosslinks.

15. The method of any of claims 10-14, wherein the type III collagen comprises: a) the amino acid sequence of SEQ ID NO: 1 or triple helical fragments thereof; b) amino acid residue 149 to amino acid residue 1221 of the amino acid sequence of SEQ ID NO: 1 or triple helical fragments thereof; c) amino acid residue 168 to amino acid residue 1196 of the amino acid sequence of SEQ ID NO:1 or triple helical fragments thereof; or d) either of a) or b) further comprising an isoleucine to proline substitution at amino acid residue 1205 of SEQ ID NO:1.

16. The method of claim 13, wherein the tissue augmentation is soft tissue augmentation and the composition is for use in a human subject.

17. The method of any of claims 10-16, wherein the composition is administered by injection and/or administered by a method selected from the group consisting of subcutaneous administration, intradermal administration, and subdermal administration.

18. The method of any of claims 10-17, wherein the method is for a) the treatment of fine lines, wrinkles and scars and pronounced lines, wrinkles and scars; b) treating or reducing transverse forehead lines, glabellar frown lines, nasolabial fold, vermilion border, periorbital lines, vertical lip lines, oral commissure, or for defining the lip border; or c) correcting contour deformities and distensible acne scars, or for treating tissue atrophy from disease or trauma or surgically-induced tissue irregularities.

19. A method for preparing an implantable collagen composition for use in tissue augmentation, the method comprising either:
a) providing a collagen as defined in claim 5, option a) and crosslinking the collagen; b) providing a collagen defined in any of claims 1-9, and preparing an aqueous solution of the collagen; or
c) providing a collagen as defined in claim 5, option a), crosslinking the collagen, and preparing an aqueous solution of the crosslinked collagen, thereby preparing an implantable collagen composition.

20. A kit useful for augmenting soft tissue, the kit comprising a composition as defined in any of claims 1-9, a syringe, and a needle.

## Patentansprüche

1. Implantierbare Zusammensetzung, die Kollagen umfasst, zur Verwendung in der Medizin, wobei das Kollagen aus Kollagen Typ III besteht und die Zusammensetzung zur Verwendung bei der Vermehrung von Gewebe vorgesehen ist.

2. Implantierbare Zusammensetzung nach Anspruch 1 zur Verwendung nach diesem Anspruch, wobei das Kollagen Typ III menschliches Kollagen Typ III oder synthetisches Kollagen Typ III ist.

3. Implantierbare Zusammensetzung nach Anspruch 1 oder 2 zur Verwendung nach diesem Anspruch, wobei die implantierbare Zusammensetzung ausgewählt ist aus der Gruppe, bestehend aus einem dermalen Füllstoff, einem Knochenersatzstoff (*bone-void filler*) und einem Zahnimplantat.

4. Implantierbare Zusammensetzung nach einem der vorangehenden Ansprüche zur Verwendung nach diesem Anspruch, wobei die Vermehrung von Gewebe eine Vermehrung von Weichgewebe oder eine Vermehrung von Hartgewebe ist.

5. Implantierbare Zusammensetzung nach einem der vorangehenden Ansprüche zur Verwendung nach diesem Anspruch, wobei das Kollagen a) frei von intermolekularen und intramolekularen Quervernetzungen; oder b) frei von endogenen Quervernetzungen ist.

6. Implantierbare Zusammensetzung nach einem der vorangehenden Ansprüche zur Verwendung nach diesem Anspruch, wobei das Kollagen Typ III Folgendes umfasst: a) die Aminosäuresequenz nach SEQ ID NO:1 oder Triplehelix-Fragmente davon; b) Aminosäurerest 149 bis Aminosäurerest 1221 der Aminosäuresequenz nach SEQ ID NO:1 oder Triplehelix-Fragmente davon; c) Aminosäurerest 168 bis Aminosäurerest 1196 der Aminosäuresequenz nach SEQ ID NO:1 oder Triplehelix-Fragmente; oder d) wobei entweder a) oder b) weiterhin eine Substitution von Isoleucin durch Prolin am Aminosäurerest 1205 nach SEQ ID NO:1 umfasst.

7. Implantierbare Zusammensetzung nach Anspruch 4 zur Verwendung nach diesem Anspruch, wobei die Vermehrung von Gewebe eine Vermehrung von Weichgewebe ist und die Zusammensetzung zur Verwendung in einem Menschen vorgesehen ist.

8. Zusammensetzung nach einem der vorangehenden Ansprüche zur Verwendung nach diesem Anspruch, wobei die Zusammensetzung zur Verabreichung durch Injektion und/oder zur Verabreichung durch ein Verfahren, das ausgewählt ist aus der Gruppe, bestehend aus subkutaner Verabreichung, intradermaler Verabreichung und subdermaler Verabreichung vorgesehen ist.

9. Zusammensetzung nach einem der vorangehenden Ansprüche, wobei die Zusammensetzung zur a) Verwendung bei der Behandlung von feinen Linien, Falten und Narben und ausgeprägten Linien, Falten und Narben; b) Verwendung beim Behandeln oder Vermindern von querverlaufenden Stimlinien, Glabella-Linien, einer Nasolabialfalte, einer Lippenrotgrenze, Periorbitallinien, vertikalen Lippenlinien, einer oralen Kommissur oder zum Definieren der Lippengrenze; oder c) Verwendung beim Korrigieren von Konturdeformationen und dehnbaren Aknenarben, oder zum Behandeln einer Gewebeatrophie als Folge einer Erkrankung oder eines Traumas oder von chirurgisch verursachten Gewebeunregelmäßigkeiten vorgesehen ist.

10. Verfahren zur Vermehrung von Gewebe, das die Verwendung einer implantierbaren Zusammensetzung umfasst, die Kollagen umfasst, wobei das Kollagen aus Kollagen Typ III besteht und das Verfahren kein Verfahren zur Behandlung des menschlichen oder tierischen Körpers durch Chirurgie oder Therapie ist.

11. Verfahren nach Anspruch 10, wobei das Kollagen Typ III menschliches Kollagen Typ III oder synthetisches Kollagen Typ III ist.

12. Verfahren nach Anspruch 10 oder 11, wobei die implantierbare Zusammensetzung ein dermaler Füllstoff ist.

13. Verfahren nach einem der Ansprüche 10 bis 12, wobei die Vermehrung von Gewebe eine Vermehrung von Weichgewebe ist.

14. Verfahren nach einem der Ansprüche 10 bis 13, wobei das Kollagen a) frei von intermolekularen und intramolekularen Quervernetzungen; oder b) frei von endogenen Quervernetzungen ist.

15. Verfahren nach einem der Ansprüche 10 bis 14, wobei das Kollagen Typ III Folgendes umfasst: a) die Aminosäuresequenz nach SEQ ID NO:1 oder Triplehelix-Fragmente davon; b) Aminosäurerest 149 bis Aminosäurerest 1221 der Aminosäuresequenz nach SEQ ID NO:1 oder Triplehelix-Fragmente davon; c) Aminosäurerest 168 bis Aminosäurerest 1196 der Aminosäuresequenz nach SEQ ID NO:1 oder Triplehelix-Fragmente davon; oder d) wobei entweder a) oder b) weiterhin eine Substitution von Isoleucin durch Prolin am Aminosäurerest 1205 nach SEQ ID NO:1 umfasst.

16. Verfahren nach Anspruch 13, wobei die Vermehrung von Gewebe eine Vermehrung von Weichgewebe ist und die Zusammensetzung zur Verwendung in einem Menschen vorgesehen ist.

17. Verfahren nach einem der Ansprüche 10 bis 16, wobei die Zusammensetzung durch Injektion verabreicht wird und/oder durch ein Verfahren verabreicht wird, das ausgewählt ist aus der Gruppe, bestehend aus subkutaner Verabreichung, intradermaler Verabreichung und subdermaler Verabreichung.

18. Verfahren nach einem der Ansprüche 10 bis 17, wobei das Verfahren a) zur Behandlung von feinen Linien, Falten und Narben und ausgeprägten Linien, Falten und Narben; b) zum Behandeln oder Vermindern von querverlaufenden Stirnlinien, Glabella-Linien, einer Nasolabialfalte, einer Lippenrotgrenze, Periorbitallinien, vertikalen Lippenlinien, einer oralen Kommissur oder zum Definieren der Lippengrenze; oder c) zum Korrigieren von Konturdeformationen und dehnbaren Aknenarben, oder zum Behandeln einer Gewebeatrophie als Folge einer Erkrankung oder eines Traumas oder von chirurgisch verursachten Gewebeunregelmäßigkeiten vorgesehen ist.

19. Verfahren zum Herstellen einer implantierbaren Kollagenzusammensetzung zur Verwendung bei einer Vermehrung von Gewebe, wobei das Verfahren Folgendes umfasst, entweder:
a) Verfügbarmachen eines Kollagens, wie in Anspruch 5, Option a) definiert, und Quervernetzen des Kollagens;
b) Verfügbarmachen eines Kollagens, wie in einem der Ansprüche 1 bis 9 definiert, und Herstellen einer wässrigen Lösung des Kollagens; oder
c) Verfügbarmachen eines Kollagens, wie in Anspruch 5, Option a) definiert, Quervernetzen des Kollagens und Herstellen einer wässrigen Lösung des quervernetzten Kollagens,
und dadurch Herstellen einer implantierbaren Kollagenzusammensetzung.

20. Kit, das zur Vermehrung von Weichgewebe nützlich ist, wobei das Kit eine Zusammensetzung wie in einem der Ansprüche 1 bis 9 definiert, eine Spritze und eine Nadel umfasst.

## Revendications

1. Composition implantable comprenant du collagène pour utilisation dans un médicament, dans laquelle le collagène consiste en du collagène de type III et la composition est pour utilisation dans l'augmentation de tissu.

2. Composition implantable selon la revendication 1 pour l'utilisation de cette revendication, dans laquelle le collagène de type III est du collagène de type III humain ou un collagène de type III synthétique.

3. Composition implantable selon la revendication 1 ou la revendication 2 pour l'utilisation de cette revendication, dans laquelle la composition implantable est choisie dans le groupe constitué par un produit de comblement dermique, un produit de comblement de vide osseux, et un implant dentaire.

4. Composition implantable selon l'une quelconque des revendications précédentes pour l'utilisation de cette revendication, dans laquelle l'augmentation de tissu est une augmentation de tissu mou ou une augmentation de tissu dur.

5. Composition implantable selon l'une quelconque des revendications précédentes pour l'utilisation de cette revendication, dans laquelle le collagène est a) exempt de réticulations intermoléculaires et intramoléculaires ; ou b) exempt de réticulations endogènes.

6. Composition implantable selon l'une quelconque des revendications précédentes pour l'utilisation de cette revendication, dans laquelle le collagène de type III comprend : a) la séquence d'acides aminés SEQ ID NO _{:} 1 ou des fragments en triple hélice de celle-ci ; b) du résidu d'acide aminé 149 au résidu d'acide aminé 1221 de la séquence d'acides aminés SEQ ID NO:1 ou des fragments en triple hélice de celle-ci ; c) du résidu d'acide aminé 168 au résidu d'acide aminé 1196 de la séquence d'acides aminés SEQ ID NO:1 ou des fragments en triple hélice de celle-ci ; ou d) l'un quelconque de a) ou b) comprenant en outre une substitution d'isoleucine par la proline au résidu d'acide aminé 1205 de SEQ ID NO:1.

7. Composition implantable selon la revendication 4 pour l'utilisation de cette revendication, dans laquelle l'augmentation de tissu est une augmentation de tissu mou et la composition est pour utilisation chez un sujet humain.

8. Composition selon l'une quelconque des revendications précédentes pour l'utilisation de cette revendication, dans laquelle la composition est pour administration par injection et/ou pour administration par un procédé choisi dans le groupe constitué par une administration sous-cutanée, une administration intradermique et une administration sous-dermique.

9. Composition selon l'une quelconque des revendications précédentes, dans laquelle la composition est pour a) utilisation dans le traitement de ridules, rides et cicatrices fins et de ridules, rides et cicatrices prononcés ; b) utilisation dans le traitement ou la réduction de ridules transversaux du front, des sillons intersourciliers, du sillon naso-génien, du vermillion de la lèvre, des ridules périorbitaux, des ridules labiaux verticaux, de la commissure de la bouche, ou pour définir le bord de la lèvre ; ou c) utilisation dans la correction de difformités de contour et de cicatrices d'acné distensibles, ou pour traiter une atrophie tissulaire due à une maladie ou un traumatisme ou des irrégularités tissulaires d'origine chirurgicale.

10. Procédé pour l'augmentation de tissu comprenant l'utilisation d'une composition implantable comprenant du collagène, dans lequel le collagène consiste en du collagène de type III et le procédé n'est pas un procédé pour le traitement du corps humain ou animal par chirurgie ou thérapie.

11. Procédé selon la revendication 10, dans lequel le collagène de type III est du collagène de type III humain ou du collagène de type III synthétique.

12. Procédé selon la revendication 10 ou la revendication 11, dans lequel la composition implantable est un produit de comblement dermique.

13. Procédé selon l'une quelconque des revendications 10 à 12, dans lequel l'augmentation de tissu est une augmentation de tissu mou.

14. Procédé selon l'une quelconque des revendications 10 à 13, dans lequel le collagène est a) exempt de réticulations intermoléculaires et intramoléculaires ; ou b) exempt de réticulations endogènes.

15. Procédé selon l'une quelconque des revendications 10 à 14, dans lequel le collagène de type III comprend : a) la séquence d'acides aminés SEQ ID NO : 1 ou des fragments en triple hélice de celle-ci ; b) du résidu d'acide aminé 149 au résidu d'acide aminé 1221 de la séquence d'acides aminés SEQ ID NO:1 ou des fragments en triple hélice de celle-ci ; c) du résidu d'acide aminé 168 au résidu d'acide aminé 1196 de la séquence d'acides aminés SEQ ID NO : 1 ou des fragments en triple hélice de celle-ci ; ou d) l'un quelconque de a) ou b) comprenant en outre une substitution d'isoleucine par la proline au résidu d'acide aminé 1205 de SEQ ID NO:1.

16. Procédé selon la revendication 13, dans lequel l'augmentation de tissu est une augmentation de tissu mou et la composition est pour utilisation chez un sujet humain.

17. Procédé selon l'une quelconque des revendications 10 à 16, dans lequel la composition est administrée par injection et/ou administrée par un procédé choisi dans le groupe constitué par une administration sous-cutanée, une administration intradermique et une administration sous-dermique.

18. Procédé selon l'une quelconque des revendications 10 à 17, dans lequel le procédé est pour a) le traitement de ridules, rides et cicatrices fins et de ridules, rides et cicatrices prononcés ; b) le traitement ou la réduction des ridules transversaux du front, des sillons intersourciliers, du sillon naso-génien, du vermillion de la lèvre, des ridules périorbitaux, des ridules labiaux verticaux, de la commissure de la bouche, ou pour définir le bord de la lèvre ; ou c) la correction de difformités de contour et de cicatrices d'acné distensibles, ou pour traiter une atrophie tissulaire due à une maladie ou un traumatisme ou des irrégularités tissulaires d'origine chirurgicale.

19. Procédé pour préparer une composition de collagène implantable pour utilisation dans l'augmentation de tissu, le procédé comprenant soit :
a) la fourniture d'un collagène tel que défini dans la revendication 5, option a) et la réticulation du collagène ; b) la fourniture d'un collagène défini dans l'une quelconque des revendications 1 à 9, et la préparation d'une solution aqueuse du collagène ; soit
c) la fourniture d'un collagène tel que défini dans la revendication 5, option a), la réticulation du collagène et la préparation d'une solution aqueuse du collagène réticulé,
de manière à préparer une composition de collagène implantable.

20. Kit utile pour l'augmentation de tissu mou, le kit comprenant une composition telle que définie dans l'une quelconque des revendications 1 à 9, une seringue, et une aiguille.
